# EUROPEAN PATENT APPLICATION

(11) **EP 2 815 648 A1**
(43) Date of publication of application: **24.12.2014**
(21) Application number: 14172752.9
(22) Date of filing: 17.06.2014
(51) Int. Cl.: A01N 37/00, C07D 231/22

(54) **Novel strobilurin-type compounds for combating phytopathogenic fungi**

(30) Priority: 18.06.2013 EP 13172460
(71) Applicant: BASF SE, 67056 Ludwigshafen (DE)
(72) Inventor: Grammenos, Wassilios, 67071 Ludwigshafen (DE); Rohrer, Sebastian Georgios, 68775 Ketsch (DE); Rheinheimer, Joachim, 67063 Ludwigshafen (DE); Winter, Christian, 67063 Ludwigshafen (DE); Escribano Cuesta, Ana, 68161 Mannheim (DE); Haden, Egon, 67346 Speyer (DE); Jabs, Thorsten, 67454 Haßloch (DE)

(57) **Abstract**

The present invention relates to the novel strobilurin-type compounds of formula I and the N-oxides and the salts thereof for combating phytopathogenic fungi, processes for preparing these compounds, to compositions comprising at least one such compound, to plant health applications, and to seeds coated with at least one such compound.

## Description

The present invention relates to the novel strobilurine type compounds of formula I and the N-oxides and the salts thereof for combating phytopathogenic fungi processes for preparing these compounds, to compositions comprising at least one such compound, to plant health applications, and to seeds coated with at least one such compound.

Qo inhibitor fungicides, often refered to as strobilurin-type fungicides (Sauter 2007: Chapter 13.2. Strobilurins and other complex III inhibitors. In: Krämer, W.; Schirmer, U. (Ed.) - Modern Crop Protection Compounds. Volume 2. Wiley-VCH Verlag 457-495), are conventionally used to control a number of fungal pathogens in crops. Qo inhibitors typically work by inhibiting respiration by binding to a ubihydroquinone oxidation center of a cytochrome bc₁ complex (electron transport complex III) in mitochondria. Said oxidation center is located on the outer side of the inner mitochrondrial membrane. A prime example of the use of Qo inhibitors includes the use of, for example, strobilurins on wheat for the control of *Septoria tritici* (also known as *Mycosphaerella graminicola*), which is the cause of wheat leaf blotch. Unfortunately, widespread use of such Qo inhibitors has resulted in the selection of mutant pathogens which are resistant to such Qo inhibitors (Gisi et al., Pest Manag Sci 56, 833-841, (2000). Resistance to Qo inhibitors has been detected in several phytopathogenic fungi such as Blumeria graminis, Mycosphaerella fijiensis, Pseudoperonspora cubensis or Venturia inaequalis. Although several resistance mechanisms have been detected meanwhile (e.g. Jabs et al. Phytomedizin 31, 15-16 (2001); Olaya et al., Pestic Sci 54, 230-236 (1998), the major part of resistance to Qo inhibtors in agricultural uses has been attributed to pathogens containing a single amino acid residue substitution G143A in the cytochrome b gene for their cytochrome bc₁ complex, the target protein of Qo inhibitors. See, for example, Lucas, Pestic Outlook 14(6), 268-70 (2003); and Fraaije et al., Phytopathol 95(8), 933-41 (2005), (which both are expressly incorporated by reference herein). Thus, new methods and compositions are desirable for controlling pathogen induced diseases in crops comprising plants subjected to pathogens that are resistant to Qo inhibitors. Furthermore, in many cases, in particular at low application rates, the fungicidal activity of the known fungicidal strobilurin analogue compounds is unsatisfactory, especially in case that a high proportion of the fungal pathogens contain a mutation in the mitochondrial cytochrome b gene conferring resistance to Qo inhibitors. Based on this, it was also an object of the present invention to provide compounds having improved activity and/or a broader activity spectrum against phytopathogenic harmful fungi.

"Qo inhibitor," as used herein, includes any substance that is capable of diminishing and/or inhibiting respiration by binding to a ubihydroquinone oxidation center of a cytochrome bc₁ complex in mitochondria. The oxidation center is typically located on the outer side of the inner mitochrondrial membrane.

From WO 2009/155095, the use of a Qi inhibitor UK2A is known for combating phytopathogenic fungi that are resistant to Qo inhibitors. Qi inhibitors typically work by inhibiting respiration by binding to a ubihydroquinone oxidation center of a cytochrome bcl complex in mitochondria, the said oxidation center being located on the inner side of the inner mitochrondrial membrane.

Certain strobilurin type compounds of formula I, wherein R⁴ is 1-methoxycarbonyl-2-methoxy-ethen-1-yl (defined as R4-2 herein, wherein X is O) and R¹ is CF₃, are mentioned in WO 1998/021174: (E)-2-[1-methoxy-meth-(E)-ylidene]-5-(4-phenoxy-phenoxy)-3-trifluoromethyl-pent-3-enoic acid methyl ester (CAS No. 207852-99-1); (E)-2-[1-methoxy-meth-(E)-ylidene]-5-(3-phenoxy-phenoxy)-3-trifluoromethyl-pent-3-enoic acid methyl ester (207853-00-7); (E)-2-[1-methoxy-meth-(E)-ylidene]-4-methyl-5-(3-phenoxy-phenoxy)-3-trifluoromethyl-pent-3-enoic acid methyl ester; and (E)-5,5,5-trifluoro-2-[1-methoxy-meth-(E)-ylidene]-3-methyl-4-(4-phenoxy-phenoxymethyl)-pent-3-enoic acid methyl ester. However, it is not mentioned that the strobilurine type compounds inhibit fungal pathogens containing a mutation in the mitochondrial cytochrome b gene conferring resistance to Qo inhibitors. II

Further, certain strobilurin type compounds, wherein R¹ and R² together with the two carbon atoms linking them form a phenyl ring and wherein R⁴ is 1-methyl-1,4-dihydro-tetrazole-5-one-4-yl (R4-7) are known inter alia from WO 1996/036229, WO 1999/046246 and DE 199 00 571 A1.

The strobilurin-analogue compounds according to the present invention differ from those described in the abovemention publications by the specific formula I and by inhibiting respiration by binding to a ubihydroquinone oxidation center of a cytochrome bcl complex in mitochondria which defines them as Qo inhibitors. Besides the strobilurin analogue-specific structural elements R⁴, these compounds contain two specific carbon atoms bound by a double bond wherein the groups R¹ and R² are cis-oriented together with the specific substituent R^{C} as defined below.

Accordingly, the present invention relates to compounds of formula I 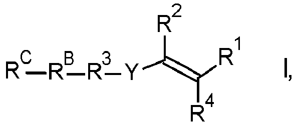 wherein:
- R¹,R²: independently of each other are hydrogen, halogen, C₁-C₄-alkyl, C₁-C₄-alkoxy, C₂-C₆-alkenyl, C₂-C₆-alkenyloxy, C₂-C₆-alkynyl, C₃-C₆-cycloalkyl or C₃-C₆-cycloalkyl-C₁-C₄-alkyl, wherein the groups R¹ and R² are cis-oriented, and wherein the aliphatic moieties of R¹ and/or R² may carry 1, 2, 3 or up to the maximum number of identical or different groups R^{a} which independently of one another are selected from:
- R^{a}: halogen, CN, nitro, C₁-C₄-alkyl, C₁-C₄-alkoxy, C₁-C₄-haloalkyl and C₁-C₄-haloalkoxy;
- Y: is a direct bond or a divalent group selected from -OCH₂-, -CH₂- or -CH₂CH₂-, where the bond depicted on the left side of the divalent group Y is attached to R³, and the bond depicted on the right side is attached to the carbon atom being substituted by R2;
- R³: is phenyl or a 3- to 10-membered saturated, partially unsaturated or aromatic mono- or bicyclic heterocyclyl wherein the ring member atoms of the heterocyclyl include besides carbon atoms 1, 2, 3 or 4 heteroatoms selected from the group of N, O and S,
wherein the group R³ may 1, 2, 3 or up to the maximum possible number of identical or different groups R^{b} which independently of one another are selected from:
- R^{b}: , which may be the same or different to any other R^{b}, is amino, halogen, hydroxyl, oxo, nitro, CN, carboxyl, C₁-C₄-alkyl, C₂-C₄-alkenyl, C₂-C₄-alkynyl, C₁-C₄-haloalkyl, C₁-C₄-alkoxy, C₁-C₄-haloalkoxy, C₃-C₆-cycloalkyl, C₃-C₆-cycloalkenyl, C₂-C₆-alkenyloxy, C₃-C₆-alkynyloxy, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₄-alkylamino, C₁-C₄-alkoxycarbonyl, C₁-C₄-alkylcarbonyl-oxy, phenyl, naphthyl or a 3- to 10-membered saturated, partially unsaturated or aromatic mono- or bicyclic heterocyclyl which, in addition to carbon atoms, contains one to four heteroatoms from the group consisting of N, O and S as ring members; and wherein the aforementioned phenyl and heterocyclyl groups R^{b} are attached via a direct bond, an oxygen or sulfur atom, and two radicals R^{b} that are bound to adjacent ring member atoms of the cyclic group R³ may form together with said ring member atoms a fused 5-, 6- or 7-membered saturated, partially unsaturated or aromatic cycle, which may be a carbocycle or heterocycle, wherein the ring member atoms of the heterocycle include besides carbon atoms 1, 2, 3 or 4 heteroatoms selected from the group of N, O and S;
- R^{B}: is phenyl or a 3- to 10-membered saturated, partially unsaturated or aromatic mono- or bicyclic heterocyclyl wherein the ring member atoms of the heterocyclyl include besides carbon atoms 1, 2, 3 or 4 heteroatoms selected from the group of N, O and S,
wherein the group R^{B} may carry 1, 2, 3 or up to the maximum possible number of identical or different groups R^{CC}:
- R^{CC},: which may be the same or different to any other R^{CC}, is halogen, hydroxyl, nitro, CN, carboxyl, C₁-C₄-alkyl, C₂-C₄-alkenyl, C₂-C₈-alkynyl, C₁-C₄-haloalkyl, C₁-C₄-alkoxy, C₁-C₄-haloalkoxy, C₁-C₆-alkoxyimino-C₁-C₄-alkyl, C₂-C₆-alkenyl-oxyimino-C₁-C₄-alkyl , C₂-C₆-alkynyloxyimino-C₁-C₄-alkyl, C₁-C₆-alkoxyimino-, C₂-C₆-alkenyloxyimino-, C₂-C₆-alkynyloxyimino-, C₂-C₆-haloalkenyloxyimino-, -SR^{D}, -S(=O)R^{D}, S(=O)₂R^{D}, -N(R^{E})₂, -NH-C(=O)-N(R^{F})₂, -O-C(R^{F})-C(R^{F})=N-O-R^{F}, C₃-C₆-cycloalkyl, C₃-C₆-cycloalkenyl, phenyl or a 5-membered saturated, partially unsaturated or aromatic heterocyclyl which, in addition to carbon atoms, contains one to three heteroatoms from the group consisting of N, O and S as ring members; wherein the aforementioned cyclic groups R^{CC} are attached via a direct bond, an oxygen or sulfur atom, and where the aliphatic or cyclic groups R^{CC} for their part may carry 1, 2, 3 or up to the maximum possible number of identical or different groups R^{d}:
- R^{d}: , which may be the same or different to any other R^{d}, is halogen, C₁-C₄-alkyl or C₁-C₄-haloalkyl;
- R^{C}: is -SR^{D}, -S(=O)R^{D}, -S(=O)₂R^{D}, -N(R^{E})₂, -NH-C(=O)-N(R^{F})₂ or -O-C(R^{F})-C(R^{F})=N-O-R^{F}, wherein
- R^{D}: is C₁-C₄-alkyl, C₁-C₄-haloalkyl, C₁-C₄-alkoxy or C₁-C₄-haloalkoxy;
- R^{E}: , which may be the same or different to any other R^{E}, is hydrogen, C₁-C₄-alkyl, C₁-C₄-haloalkyl, C₁-C₄-alkylcarbonyl or C₁-C₄-haloalkylcarbonyl;
- R^{F}: , which may be the same or different to any other R^{F}, is hydrogen, C₁-C₄-alkyl or C₁-C₄-haloalkyl;
- R⁴: is a monovalent group selected from formulae R4-1 to R4-7 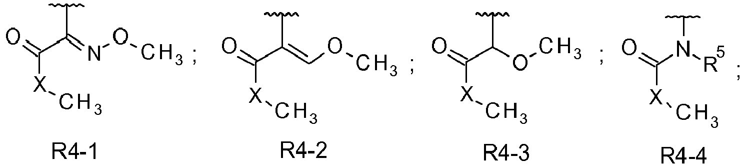 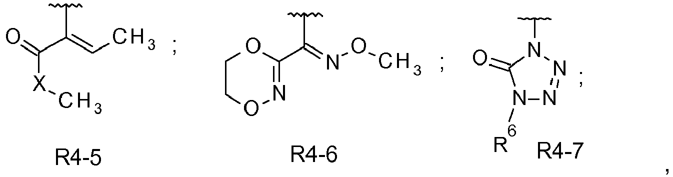 wherein the jagged line defines the point of attachment, and
X is a direct bond or a divalent group CH₂, O or NH,
R⁵ is C₁-C₄-alkyl, C₁-C₄-alkoxy, C₁-C₄-haloalkyl, C₁-C₄-haloalkoxy or C₃-C₆-cycloalkyl; and
R⁶ is C₁-C₄-alkyl or C₁-C₄-haloalkyl;
and the N-oxides and the agriculturally acceptable salts thereof.

Furthermore, the invention also relates to the use of compounds of formula I for combating phytopathogenic fungi, preferably of phytopathogenic fungi containing a mutation in the mitochondrial cytochrome b gene conferring resistance to Qo inhibitors

Furthermore, the present invention also relates to methods for combating phytopathogenic fungi, preferably for combating phytopathogenic fungi containing a mutation in the mitochondrial cytochrome b gene conferring resistance to Qo inhibitors using the abovementioned compounds of formula I.

The term "compounds I" refers to compounds of formula I. Likewise, this terminology applies to all sub-formulae, e. g. "compounds I.2" refers to compounds of formula I.2 or "compounds V" refers to compounds of formula V, etc..

The compounds I can be obtained by various routes in analogy to prior art processes known (e.g. WO 1998/021174, J Organomet Chem 689, 575-584 (2004), WO 1996/036229) and, advantageously, by the synthesis shown in the following schemes and in the experimental part of this application.

A suitable method to prepare compounds I is illustrated in scheme 1. It starts with the reduction of an acetylene compound II with a reducing agent like lithium aluminium hydride preferably in the presence of a solvent. Suitable solvents are inert against the reducing agent used and preferably selected from cyclic or aliphatic ethers like dietyl ether, tetrahydrofurane (THF), 1,4-dioxane, and methyl-tert.-butyl ether (MTBE). The reaction temperature can be between -40°C and 100°C, preferably -20 to 60°C. After a reduced intermediate has been formed, a tin compound of formula III, wherein Alk defines a suitable alkyl residue like methyl, ethyl, n-propyl, or n-butyl and wherein L is a leaving group such as halogen, ethoxy and methoxy, in particular methoxy, is added. The resulting intermediate IV is a stable compound which can be isolated and purified with the usual methods (for example extraction and chromatography).

Compound IV is further reacted with compound V to yield the intermediate VI applying the usual methods for coupling aliphatic alcohols with hydroxyl compounds IV. The Mitsonobu reaction has proven especially useful.

Compound VI is then coupled with compound VII, wherein LG is a leaving group, preferably being halogen (except fluoro) or a sulfonyloxy group such as triflate, preferably in the presence of a suitable catalyst such as known transition metal catalysts, more preferably palladium catalalysts, wherein the ligand may ber trifuryl phoshine, triphenyl phosphine, tritolyl phosphine or bidentate phosphine ligands. The. Copper compounds, such as CuI₂ can be added to improve the reaction. A wide variety of solvents is possible here, with THF, 1,4-dioxane and amides like dimethylformamide (DMF) being prefered. The reaction temperatures can be -20 to 150°C, preferably 20 to 120°C.

The resulting compounds I, wherein Y is -OCH₂- and R² is H, can be further modified. For example, if R⁴ contains an ester group, VIII can be transformed into a methyl amide by reaction with methyl amine.

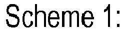

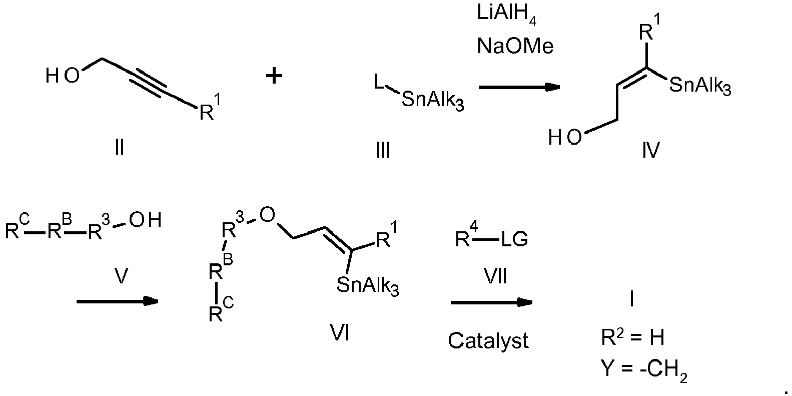

Another general method to prepare compounds I is illustrated in scheme 2. The starting materials VIII are either known or can be prepared analogous to known compounds. The Wittig-Horner reaction of compounds VIII with compounds IX illustrated here (see also Tetrahedron Lett. 1988, 29, 3361) can be replaced by the Wittig reaction if this results in better yields. These reactions as well as the reaction conditions are well known. A specific problem is the E/Z-ratio in the newly formed double bond. The desired isomer is usually accompanied by some undesired isomer, which has to be removed by purification known in the art (e. g. chromatography, destillation, crystallization, etc.).

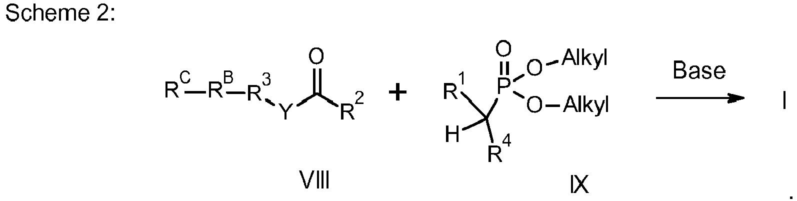

A route to compounds I, wherein R⁴ is of formula R4-4, is illustrated in scheme 3. The compound X can be obtained for example from compound VI by direct reaction with iodine. The iodine in compounds X may be replaced by other suitable leaving groups, for example by bromine, chlorine or triflate. The sodium atom in the salt VII can be replaced by other suitable metal atoms, for example potassium, lithium, magnesium, calcium, etc.. The coupling reaction of X and XI is performed preferably in the presence of a transition metal catalyst being preferably copper in the presence of a nitrogen containing ligand system (see e.g.: Tetrahedron Lett 2008, 49 (26), 4196-4199; Org Lett. 2004, 6 (11), 1809-1812).

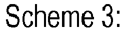

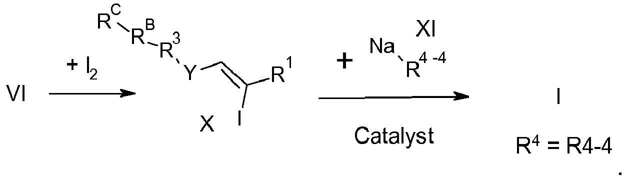

A route to 3-hydroxy pyrazole compounds V, wherein R³ is pyrazolyl, is illustrated in scheme 4. The compound XII can be obtained for example from cyclic amine compound XI (preferably R^{B} is phenyl) by reaction with NaNO₂ and SnCl₂. Then, compound XII can be reacted with ethyl prop-2- enoate to obtain the pyrazolidin-3-one compound XIII. Compound XIII can be oxidized in presence of CuCl to the 3-hydroxy-pyrazole compound V. Several oxidizing agents and procedures are known in the art to obtain compounds V: e.g. oxygen in presence of a base (WO 1998/027062), elemental sulfur (J. Gen. Chem. USSR, Engl. Trans. 31, 1770 (1961)), elemental halogens (Chem. Heterocycl. Comp. 5, 527 (1969); J. Prakt. Chem. 318, 253 (1976); J. Prakt. Chem. 313, 1118 (1971)), peroxides (J. Med. Chem. 34, 1560 (1991); DE-A 34 15 385), atmospheric oxygen (J. Prakt. Chem. 313, 115 (1971); J. Prakt. Chem 313, 1118 (1971)), and atmnospheric oxygen in presence of metal salts, preferably Fe and Cu salts of the following oxidation numbers: Fe-II, Fe-III, Cu-I and Cu-II salts (WO 1997/03969).

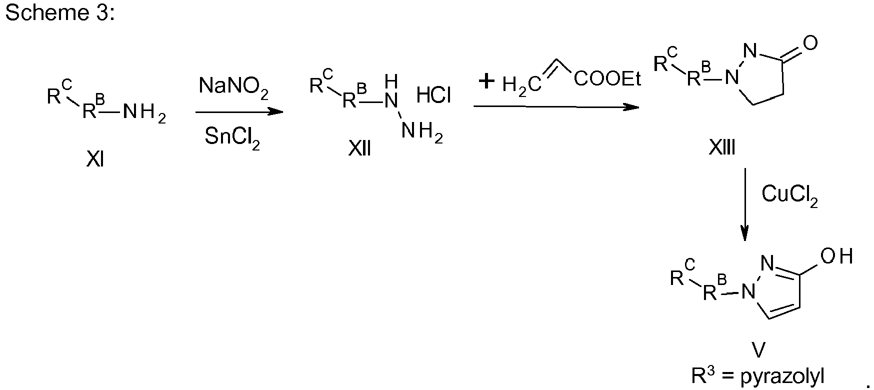

If individual compounds I cannot be obtained by the routes described above, they can be prepared by derivatization of other compounds I.

If the synthesis yields mixtures of isomers in the case of oximes, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl (isobutyl), 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₁-C₄-alkyl" refers to a straight-chained or branched alkyl group having 1 to 4 carbon atoms.

The term "C₁-C₄-haloalkyl" refers to a straight-chained or branched alkyl group having 1 to 4 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. Likewise, the term "C₁-C₆-haloalkyl" refers to a straight-chained or branched alkyl group having 1 to 6 carbon atoms.

The term "C₁-C₆-alkoxy" refers to a straight-chain or branched alkyl group having 1 to 6 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methyl¬propoxy, 2-methylpropoxy or 1,1-dimethylethoxy, O(CH₂)₄CH₃ or O(CH₂)₅CH₃. Likewise, the term "C₁-C₄-alkoxy" refers to a straight-chain or branched alkyl group having 1 to 4 carbon atoms which is bonded via an oxygen, at any position in the alkyl group.

The term "C₁-C₄-haloalkoxy" refers to a C₁-C₄-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, 2-fluoropropoxy, 3-fluoropropoxy, 2,2-difluoropropoxy, 2,3-difluoro¬propoxy, 2-chloropropoxy, 3-chloropropoxy, 2,3-dichloropropoxy, 2-bromo¬propoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromo¬ethoxy, 4-fluorobutoxy, 4-chlorobutoxy, 4-bromobutoxy or nonafluorobutoxy. Likewise, the term "C₁-C₆-haloalkoxy" refers to a C₁-C₆-alkoxy group as defined above, wherein some or all of the hydrogen atoms may be replaced by halogen atoms as mentioned above.

The term "C₁-C₄-alkoxy-C₁-C₄-alkyl" refers to alkyl having 1 to 4 carbon atoms, wherein one hydrogen atom of the alkyl radical is replaced by a C₁-C₄-alkoxy group. Likewise, the term "C₁-C₆-alkoxy-C₁-C₆-alkyl" refers to alkyl having 1 to 6 carbon atoms, wherein one hydrogen atom of the alkyl radical is replaced by a C₁-C₆-alkoxy group.

The term "C₁-C₄-alkylamino" refers to an amino radical carrying one C₁-C₄-alkyl group as substituent, e.g. methylamino, ethylamino, propylamino, 1-methylethylamino, butylamino, 1-methylpropylamino, 2-methylpropylamino, 1,1-dimethylethylamino and the like. Likewise, the term "C₁-C₆-alkylamino" refers to an amino radical carrying one C₁-C₆-alkyl group as substituent.

The term "di(C₁-C₄-alkyl)amino" refers to an amino radical carrying two identical or different C₁-C₄-alkyl groups as substituents, e. g. dimethylamino, diethylamino, di-n-propylamino, diisopropylamino, N-ethyl-N-methylamino, N-(n-propyl)-N-methylamino, N-(isopropyl)-N methylamino, N-(n-butyl)-N-methylamino, N-(2-butyl)-N methylamino, N-(isobutyl)-N-methylamino, and the like. Likewise, the term "di(C₁-C₆-alkyl)amino" refers to an amino radical carrying two identical or different C₁-C₆-alkyl groups as substituents.

The term "C₁-C₄-alkoxyimino" refers to a divalent imino radical (C₁-C₄-alkyl-O-N=) carrying one C₁-C₄-alkoxy group as substituent, e.g. methylimino, ethylimino, propylimino, 1-methylethylimino, butylimino, 1-methylpropylimino, 2-methylpropylimino, 1,1-dimethylethylimino and the like.

The term "C₁-C₆-alkoxyimino-C₁-C₄-alkyl" refers to alkyl having 1 to 4 carbon atoms (as defined above), wherein two hydrogen atoms of one carbon atom of the alkyl radical are replaced by a divalent C₁-C₆-alkoxyimino radical (C₁-C₆-alkyl-O-N=) as defined above.

The term "C₂-C₆-alkenyloxyimino-C₁-C₄-alkyl" refers to alkyl having 1 to 4 carbon atoms (as defined above), wherein two hydrogen atoms of one carbon atom of the alkyl radical are replaced by a divalent C₂-C₆-alkenyloxyimino radical (C₂-C₆-alkenyl-O-N=).

The term "C₂-C₆-alkynyloxyimino-C₁-C₄-alkyl" refers to alkyl having 1 to 4 carbon atoms (as defined above), wherein two hydrogen atoms of one carbon atom of the alkyl radical are replaced by a divalent C₂-C₆-alkynyloxyimino radical (C₂-C₆-alkynyl-O-N=).

The term "C₁-C₄-alkylcarbonyl" refers to a C₁-C₄-alkyl radical which is attached via a carbonyl group. The term "(C₁-C₆-alkoxy)carbonyl" refers to a C₁-C₆-alkoxy radical which is attached via a carbonyl group.

The term "C₁-C₆-alkylaminocarbonyl" refers to a C₁-C₆-alkylamino radical which is attached via a carbonyl group. Likewise, the term "di(C₁-C₆-alkyl)aminocarbonyl" refers to a di(C₁-C₆)alkylamino radical which is attached via a carbonyl group.

The term "C₂-C₄-alkenyl" refers to a straight-chain or branched unsaturated hydrocarbon radical having 2 to 4 carbon atoms and a double bond in any position, e.g. ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl. Likewise, the term "C₂-C₆-alkenyl" refers to a straight-chain or branched unsaturated hydrocarbon radical having 2 to 6 carbon atoms and a double bond in any position.

The term "C₂-C₄-alkynyl" refers to a straight-chain or branched unsaturated hydrocarbon radical having 2 to 4 carbon atoms and containing at least one triple bond, such as ethynyl, 1-propynyl, 2-propynyl, 1-butynyl, 2-butynyl, 3-butynyl, 1-methyl-2-propynyl. Likewise, the term "C₂-C₆-alkynyl" refers to a straight-chain or branched unsaturated hydrocarbon radical having 2 to 6 carbon atoms and at least one triple bond.

The term "C₃-C₆-cycloalkyl" refers to monocyclic, bicyclic, saturated hydrocarbon radicals having 3 to 6 carbon ring members, such as cyclopropyl, cyclobutyl, cyclopentyl or cyclohexyl.

Likewise, the term "C₃-C₆-cycloalkenyl" refers to unsaturated hydrocarbon radicals having 3 to 6 carbon ring members and a double bond in any position, such as cyclopropenyl, cyclobutenyl, cyclopentenyl or cyclohexenyl.

The term "C₃-C₆-cycloalkyl-C₁-C₄-alkyl" refers to alkyl having 1 to 4 carbon atoms (as defined above), wherein one hydrogen atom of the alkyl radical is replaced by a cycloalkyl radical having 3 to 6 carbon atoms.

The term "phenyl-C₁-C₄-alkyl" refers to alkyl having 1 to 4 carbon atoms (as defined above), wherein one hydrogen atom of the alkyl radical is replaced by a phenyl radical.

The term "the group R³ may carry 1, 2, 3 or up to the maximum possible number of identical or different groups R^{b"} refers to a cyclic group R³ as defined herein which is bound to a cyclic group R^{B} as defined herein and and to a linker Y as defined herein, wherein the group R³ optionally carries 1, 2, 3 or up to the maximum possible number of identical or different groups R^{b} as defined herein.

Likewise, the term "group R^{B} may carry 1, 2, 3 or up to the maximum possible number of identical or different groups R^{CC"} refers to a cyclic group R^{B} as defined herein which is bound to a group R^{C} as defined herein and and to a cyclic group R³ as defined herein, wherein the group R^{B} optionally carries 1, 2, 3 or up to the maximum possible number of identical or different groups R^{CC} as defined herein.

The term -SR^{D} refers to a sulfanyl group. The term -S(=O)R^{D} refers to a sulfinyl group. Ther S(=O)₂R^{D} refers to a sulfonyl group.

Agriculturally acceptable salts of compounds I encompass especially the salts of those cations or the acid addition salts of those acids whose cations and anions, respectively, have no adverse effect on the fungicidal action of the compounds I. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting a compound of formula I with an acid of the corresponding anion, preferably of hydrochloric acid, hydrobromic acid, sulfuric acid, phosphoric acid or nitric acid.

The compounds of formula I can be present in atropisomers arising from restricted rotation about a single bond of asymmetric groups. They also form part of the subject matter of the present invention.

Depending on the substitution pattern, the compounds of formula I and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In respect of the the variables, the embodiments of the intermediates correspond to the embodiments of the compounds I.

Preference is given to those compounds I and where applicable also to compounds of all sub-formulae provided herein, e. g. formulae I.1 and I.2, and to the intermediates such as compounds II, III, IV and V, wherein the substituents and variables (such as R¹, R², R³, R⁴, R⁵, R⁶, X, Y, R^{B}, R^{C}, R^{a}, R^{b} R^{c}, R^{d}, R^{CC}, R^{D}, R^{E} and R^{F}) have independently of each other or more preferably in combination (any possible combination of 2 or more substituents as defined herein) the following meanings:
One embodiment of the invention relates to compounds I, wherein R¹ is halogen, C₁-C₄-alkyl, C₁-C₄-alkoxy, C₂-C₆-alkenyl, C₂-C₆-alkenyloxy, C₂-C₆-alkynyl, C₃-C₆-cycloalkyl or C₃-C₆-cycloalkyl-C₁-C₄-alkyl, wherein the aliphatic moieties of R¹ may carry 1, 2, 3 or up to the maximum number of identical or different groups R^{b} which independently of one another are selected from halogen, CN, nitro, C₁-C₄-alkyl, C₁-C₄-alkoxy, C₁-C₄-haloalkyl and C₁-C₄-haloalkoxy; more preferably R¹ is halogen, C₁-C₄-alkyl, C₁-C₄-haloalkyl, C₁-C₄-alkoxy, C₁-C₄-alkoxy-C₁-C₄-alkyl or C₁-C₄-haloalkoxy-C₁-C₄-alkyl; even more preferably R¹ is halogen, C₁-C₄-alkyl, C₁-C₄-chloroalkyl, C₁-C₄-alkoxy, C₁-C₄-alkoxy-C₁-C₄-alkyl or C₁-C₄-haloalkoxy-C₁-C₄-alkyl; in particular R¹ is C₁-C₄-alkyl.

According to a further embodiment, R² is hydrogen, halogen, C₁-C₄-alkyl, C₁-C₄-haloalkyl, C₁-C₄-alkoxy, C₁-C₄-alkoxy-C₁-C₄-alkyl or C₁-C₄-haloalkoxy-C₁-C₄-alkyl; more preferably R² is hydrogen, halogen, C₁-C₄-alkyl, C₁-C₄-alkoxy, or C₁-C₄-alkoxy-C₁-C₄-alkyl; even more preferably R² is hydrogen, C₁-C₄-alkyl or C₁-C₄-alkoxy; in particular hydrogen.

According to a further embodiment, R² is halogen, C₁-C₄-alkyl, C₁-C₄-alkoxy, C₂-C₆-alkenyl, C₂-C₆-alkenyloxy, C₂-C₆-alkynyl, C₃-C₆-cycloalkyl or C₃-C₆-cycloalkyl-C₁-C₄-alkyl, wherein the aliphatic moieties of R² may carry 1, 2, 3 or up to the maximum number of identical or different groups R^{a} which independently of one another are selected from halogen, CN, nitro, C₁-C₄-alkyl, C₁-C₄-alkoxy, C₁-C₄-haloalkyl and C₁-C₄-haloalkoxy.

Further embodiments relate to compounds I in which R¹ and R² are one of the following combinations R-1 to R-36 in Table R:

**Table R:**

| **No.** | **R¹** | **R²** | | **No.** | **R¹** | **R²** |
|---|---|---|---|---|---|---|
| R-1 | H | H | | R-20 | F | CH₃ |
| R-2 | H | CH₃ | | R-21 | F | CH₂CH₃ |
| R-3 | H | CH₂CH₃ | | R-22 | F | F |
| R-4 | H | F | | R-23 | F | Cl |
| R-5 | H | Cl | | R-24 | F | OCH₃ |
| R-6 | H | OCH₃ | | R-25 | Cl | H |
| R-7 | CH₃ | H | | R-26 | Cl | CH₃ |
| R-8 | CH₃ | CH₃ | | R-27 | Cl | CH₂CH₃ |
| R-9 | CH₃ | CH₂CH₃ | | R-28 | Cl | F |
| R-10 | CH₃ | F | | R-29 | Cl | Cl |
| R-11 | CH₃ | Cl | | R-30 | Cl | OCH₃ |
| R-12 | CH₃ | OCH₃ | | R-31 | OCH₃ | H |
| R-13 | CH₂CH₃ | H | | R-32 | OCH₃ | CH₃ |
| R-14 | CH₂CH₃ | CH₃ | | R-33 | OCH₃ | CH₂CH₃ |
| R-15 | CH₂CH₃ | CH₂CH₃ | | R-34 | OCH₃ | F |
| R-16 | CH₂CH₃ | F | | R-35 | OCH₃ | Cl |
| R-17 | CH₂CH₃ | Cl | | R-36 | OCH₃ | OCH₃ |
| R-18 | CH₂CH₃ | OCH₃ | | | | |
| R-19 | F | H | | | | |

According to a further embodiment, Y is a divalent group selected from -OCH₂-, -CH₂-, -CH₂CH₂-, where the bond depicted on the left side of the divalent group Y is attached to R³, and the bond depicted on the right side is attached to the carbon atom being substituted by R²; in particular Y is -OCH₂, which compounds are of formula I.1:

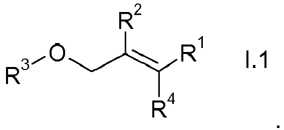

According to a further embodiment, Y is -CH₂-, which compounds are of formula I.2:

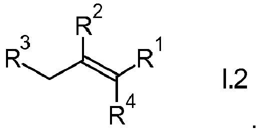

According to a further embodiment, Y is -CH₂CH₂-, which compounds are of formula I.3:

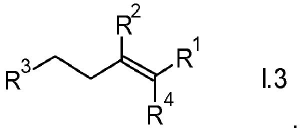

Particularly preferred embodiments of the invention relate to compounds I, wherein the group Y is one of the following radicals Y-1 to Y-6, where the bond depicted on the left side of the divalent group Y is attached to R³, and the bond depicted on the right side is attached to the carbon atom being substituted by R²:

| **No.** | **Y** | | **No.** | **Y** |
|---|---|---|---|---|
| Y-1 | -OCH₂- | | Y-3 | -CH₂CH₂- |
| Y-2 | -CH₂- | | | |

Particularly preferred embodiments of the invention relate to compounds I, wherein the group Y is -OCH₂-.

According to a further embodiment, R³ is phenyl or a 3- to 10-membered saturated, partially unsaturated or aromatic mono- or bicyclic heterocyclyl wherein the ring member atoms of the heterocyclyl include besides carbon atoms 1, 2, 3 or 4 heteroatoms selected from the group of N, O and S, wherein R³ may carry 1, 2, 3 or up to the maximum possible number of identical or different groups R^{b} as defined herein; more preferably said 3- to 10-membered saturated, partially unsaturated or aromatic mono- or bicyclic heterocyclyl is a 5- to 6-membered heteroaryl wherein the ring member atoms of the heteroaryl include besides carbon atoms 1, 2 or 3 heteroatoms selected from the group of N, O and S.

According to a further embodiment, R³ is phenyl, wherein the phenyl may carry 1, 2, 3 or up to the maximum possible number of identical or different groups R^{b} as defined herein.

According to a further embodiment, R³ is phenyl, wherein the phenyl does not carry any group R^{b} as defined herein.

According to a further embodiment, R³ is a 5-membered-heteroaryl, wherein the ring member atoms of the heterocyclyl include besides carbon atoms 1, 2 or 3 heteroatoms selected from the group of N, O and S, wherein the heteroaryl may carry 1, 2, 3 or up to the maximum possible number of identical or different groups R^{b} as defined herein; more preferably said heteroaryl is pyrazolyl, imidazolyl, oxazolyl, isoxazolyl, thiazolyl, 1,2,4-triazolyl, 1,2,4-oxadiazolyl, 1,3,4-oxadiazolyl or 1,2,4-thiadiazolyl.

According to a further embodiment, R³ is a pyrazolyl or 1,2,4-triazolyl.

According to a further embodiment, R³ is a pyrazol-3-yl or 1,2,4-triazol-3-yl, wherein R³ is connected to the linker Y in position 3.

According to a further embodiment, R³ is a pyrazolyl or 1,2,4-triazolyl, wherein R³ does not carry any R^{b} groups.

According to a further embodiment, R³ is a pyrazol-3-yl or 1,2,4-triazol-3-yl, wherein R³ is connected to the linker Y in position 3, wherein R³ does not carry any R^{b} groups.

According to a further embodiment, R³ is pyrazolyl, imidazolyl, oxazolyl, isoxazolyl, thiazolyl, 1,2,4-triazolyl, 1,2,4-oxadiazolyl, 1,3,4-oxadiazolyl or 1,2,4-thiadiazolyl and R^{B} is phenyl, wherein said phenyl may carry 1, 2, 3 or up to the maximum possible number of identical or different groups R^{CC}, which may be the same or different to any other R^{CC}, wherein R^{CC} is halogen, C₁-C₄-alkyl, C₂-C₄-alkenyl, C₁-C₄-haloalkyl, C₁-C₄-alkoxy, C₁-C₄-haloalkoxy, C₁-C₄-alkoxyimino- C₁-C₄-alkyl, phenyl or a 5-membered saturated, partially unsaturated or aromatic heterocyclyl which, in addition to carbon atoms, contains one to three heteroatoms from the group consisting of N, O and S as ring members; and wherein the aforementioned heterocyclyl groups R^{CC} are attached via a direct bond, an oxygen or sulfur atom and for their part may carry 1, 2, 3 or up to the maximum possible number of identical or different groups R^{d} as defined herein.

According to a further embodiment, R³ is pyrazol-3-yl or 1,2,4-triazol-3-yl, wherein R³ does not carry any R^{b} groups, and R^{B} is phenyl, wherein said phenyl may carry 1, 2, 3 or up to the maximum possible number of identical or different groups R^{CC}, which may be the same or different to any other R^{CC}, wherein R^{CC} is halogen, C₁-C₄-alkyl, C₂-C₄-alkenyl, C₁-C₄-haloalkyl, C₁-C₄-alkoxy, C₁-C₄-haloalkoxy, C₁-C₄-alkoxyimino-C₁-C₄-alkyl, phenyl or a 5-membered saturated, partially unsaturated or aromatic heterocyclyl which, in addition to carbon atoms, contains one to three heteroatoms from the group consisting of N, O and S as ring members; and wherein the aforementioned heterocyclyl groups R^{c} are attached via a direct bond, an oxygen or sulfur atom and for their part may carry 1, 2, 3 or up to the maximum possible number of identical or different groups R^{d} as defined herein.

According to a further embodiment, R³ is a 6-membered-heteroaryl, wherein the ring member atoms of the heterocyclyl include besides carbon atoms 1, 2 or 3 heteroatoms selected from the group of N, O and S, wherein the heteroaryl may carry 1,2,3,4 or up to the maximum possible number of identical or different groups R^{b} as defined herein; more preferably said heteroaryl is pyridinyl or pyrimidinyl.

According to a further embodiment, R³ carries 1 or 2 identical or different groups R^{b}.

According to a further embodiment, R^{b} independently of one another are selected from carboxyl, C₁-C₄-alkyl, C₂-C₄-alkenyl, C₂-C₄-alkynyl, C₁-C₄-haloalkyl, C₁-C₄-alkoxy, C₁-C₄-haloalkoxy, C₁-C₄-alkylamino, C₁-C₄-alkoxycarbonyl, C₁-C₄-alkylcarbonyloxy, phenyl, naphthyl or a 3- to 10-membered saturated, partially unsaturated or aromatic mono- or bicyclic heterocyclyl or which, in addition to carbon atoms, contains one to four heteroatoms from the group consisting of O, N and S as ring members; and wherein the aforementioned phenyl and heterocyclyl groups R^{b} are attached via a direct bond, an oxygen or sulfur atom.

According to a further embodiment, R^{B} is phenyl, wherein said phenyl carries the group R^{C} in para- or meta-position (4- or 3-position).

According to a further embodiment, the cyclic groups R^{B} for their part carry 1, 2, 3 or up to the maximum possible number of identical or different groups R^{CC}, which, may be the same or different to any other R^{CC}, selected from halogen, hydroxyl, nitro, CN, carboxyl, C₁-C₄-alkyl, C₂-C₄-alkenyl, C₂-C₈-alkynyl, C₁-C₄-haloalkyl, C₁-C₄-alkoxy and C₁-C₄-haloalkoxy; more preferably R^{B} for their part carry 1 or 2 identical or different groups R^{c}.

According to a further embodiment, R^{CC}, which, may be the same or different to any other R^{CC}, is halogen, C₁-C₄-alkyl, C₁-C₄-haloalkyl, C₁-C₄-alkoxyimino- C₁-C₄-alkyl, phenyl or a 5-membered saturated, partially unsaturated or aromatic heterocyclyl which, in addition to carbon atoms, contains one to three heteroatoms from the group consisting of N, O and S as ring members; wherein the aforementioned cyclic groups R^{c} are attached via a direct bond, an oxygen or sulfur atom and for their part may carry 1, 2, 3 or up to the maximum possible number of identical or different groups R^{d} as defined herein.

According to a further embodiment, two radicals R^{b} that are bound to adjacent ring member atoms of the cyclic group R³ form together with said ring member atoms a fused 5-, 6- or 7-membered saturated, partially unsaturated or aromatic cycle, which may be a carbocycle or heterocycle, wherein the ring member atoms of the fused heterocycle include besides carbon atoms 1, 2, 3 or 4 heteroatoms selected from the group of N, O and S.

According to a further embodiment, R^{C} is -SR^{D}, -S(=O)R^{D} or -S(=O)₂R^{D}, wherein R^{D} is C₁-C₄-alkyl, C₁-C₄-haloalkyl, C₁-C₄-alkoxy or C₁-C₄-haloalkoxy.

According to a further embodiment, R^{C} is -SR^{D}, wherein R^{D} is C₁-C₄-alkyl or C₁-C₄-haloalkyl, in particular R^{C} is trifluoromethylsulfanyl.

According to a further embodiment, R^{C} is -N(R^{E})₂, wherein R^{E}, which may be the same or different to any other R^{E}, is hydrogen, C₁-C₄-alkyl, C₁-C₄-haloalkyl, C₁-C₄-alkylcarbonyl or C₁-C₄-haloalkylcarbonyl.

According to a further embodiment, R^{C} is -NH-C(=O)-N(R^{F})₂, wherein R^{F}, which may be the same or different to any other R^{F}, is hydrogen, C₁-C₄-alkyl or C₁-C₄-haloalkyl.

According to a further embodiment, R^{C} is -O-C(R^{F})-C(R^{F})=N-O-R^{F}, wherein R^{F}, which may be the same or different to any other R^{F}, is hydrogen, C₁-C₄-alkyl or C₁-C₄-haloalkyl

According to a further embodiment, R³ is pyrazol-3-yl or 1,2,4-triazol-3-yl, wherein R³ does not carry any R^{b} groups, and R^{B} is phenyl, wherein said phenyl carries the group R^{C} as defined herein in para or meta-position (4- or 3-position) and may carry 1, 2, 3 or 4 of identical or different groups R^{CC}, which may be the same or different to any other R^{CC}, as defined herein.

According to a further embodiment, R⁴ is R4-1 as defined herein, wherein X is O.

According to a further embodiment, R⁴ is R4-1 as defined herein, wherein X is NH.

According to a further embodiment, R⁴ is R4-2 as defined herein, wherein X is O.

According to a further embodiment, R⁴ is R4-2 as defined herein, wherein X isNH.

According to a further embodiment, R⁴ is R4-3 as defined herein, wherein X is O.

According to a further embodiment, R⁴ is R4-3 as defined herein, wherein X is NH.

According to a further embodiment, R⁴ is R4-4 as defined herein, wherein X is O.

According to a further embodiment, R⁴ is R4-4 as defined herein, wherein X is NH.

According to a further embodiment, R⁵ is C₁-C₄-alkyl, C₁-C₄-haloalkyl, C₁-C₄-alkoxy or C₁-C₄-haloalkoxy, more preferably C₁-C₂-alkyl or C₁-C₂-alkoxy, even more preferably methyl, ethyl or methoxy.

According to a further embodiment, R⁴ is R4-4 as defined herein, wherein X is O and wherein R⁵ is methyl, ethyl or methoxy.

According to a further embodiment, R⁴ is R4-5 as defined herein, wherein X is O.

According to a further embodiment, R⁴ is R4-5 as defined herein, wherein X is NH.

According to a further embodiment, R⁴ is R4-6 as defined herein.

According to a further embodiment, R⁴ is R4-7 as defined herein.

Further embodiments of the invention relate to compounds I, wherein the group R⁴ is one of the following radicals R4-10 to R4-45, wherein R4-1 to R4-7 are as defined above herein:

**Table R4:**

| **Line** | **R⁴** | | **Line** | **R⁴** |
|---|---|---|---|---|
| R4-10 | R4-1; X = O | | R4-29 | R4-4; X = direct bond; R⁵ = CH₃ |
| R4-11 | R4-1; X = NH | | | |
| R4-12 | R4-1; X = CH₂ | | R4-30 | R4-4; X = O; R⁵ = CH₂CH₃ |
| R4-13 | R4-1; X = direct bond | | R4-31 | R4-4; X = NH; R⁵ = CH₂CH₃ |
| R4-14 | R4-2; X = O | | R4-32 | R4-4; X = CH₂; R⁵ = CH₂CH₃ |
| R4-15 | R4-2; X = NH | | R4-33 | R4-4; X = direct bond; R⁵ = CH₂CH₃ |
| R4-16 | R4-2; X = CH₂ | | | |
| R4-17 | R4-2; X = direct bond | | R4-34 | R4-4; X = O; R⁵ = CHF₂ |
| R4-18 | R4-3; X = O | | R4-35 | R4-4; X = NH; R⁵ = CHF₂ |
| R4-19 | R4-3; X = NH | | R4-36 | R4-4; X = CH₂; R⁵ = CHF₂ |
| R4-20 | R4-3; X = CH₂ | | R4-37 | R4-4; X = direct bond; R⁵ = CHF₂ |
| R4-21 | R4-3; X = direct bond | | R4-38 | R4-5; X = O |
| R4-22 | R4-4; X = O; R⁵ = OCH₃ | | R4-39 | R4-5; X = NH |
| R4-23 | R4-4; X = NH; R⁵ = OCH₃ | | R4-40 | R4-5; X = CH₂ |
| R4-24 | R4-4; X = CH₂; R⁵ = OCH₃ | | R4-41 | R4-5; X = direct bond |
| R4-25 | R4-4; X = direct bond; R⁵ = OCH₃ | | R4-42 | R4-6 |
| R4-26 | R4-4; X = O; R⁵ = CH₃ | | R4-43 | R4-7; R⁶ = CH₃ |
| R4-27 | R4-4; X = NH; R⁵ = CH₃ | | R4-44 | R4-7; R⁶ = CH₂CH₃ |
| R4-28 | R4-4; X = CH₂; R⁵ = CH₃ | | R4-45 | R4-7; R⁶ = CHF₂ |

Further embodiments of the invention relate to compounds I, wherein the moiety R^{C}-R^{B}-R³ is one of the following radicals R3-A to R3-B, wherein # indicates the point of attachment to the linker moiety Y:

| **Line** | **R^{C}-R^{B}-R³** | | **Line** | **R^{C}-R^{B}-R³** |
|---|---|---|---|---|
| R3-A | 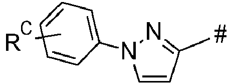 | | R3-B | 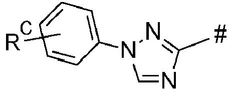 |

Further embodiments of the invention relate to compounds I, wherein the moiety R^{C}-R^{B}-R³ is one of the following radicals R3-C to R3-F, wherein # indicates the point of attachment to the linker moiety Y:

| **Line** | **R^{C}-R^{B}-R³** | | **Line** | **R^{C}-R^{B}-R³** |
|---|---|---|---|---|
| R3-C | 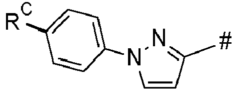 | | R3-E | 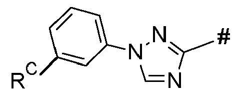 |
| R3-D | 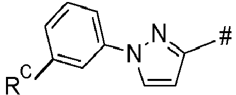 | | R3-F | 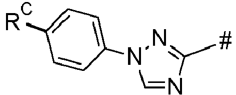 |

Particularly preferred embodiments of the invention relate to compounds I, wherein the moiety R^{C}-R^{B}-R³ is one of the following radicals R3-1 to R3-140, wherein # indicates the point of attachment to the linker moiety Y:

**Table A.**

| **Line** | **R^{C}-R^{B}-R³** | | **Line** | **R^{C}-R^{B}-R³** |
|---|---|---|---|---|
| R3-1 | 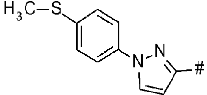 | | R3-6 | 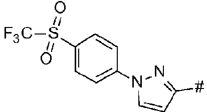 |
| R3-2 | 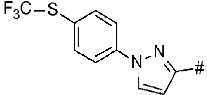 | | R3-7 | 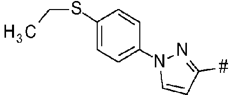 |
| R3-3 | 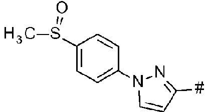 | | R3-8 | 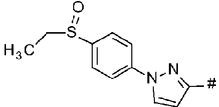 |
| R3-4 | 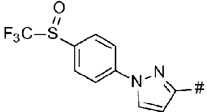 | | R3-9 | 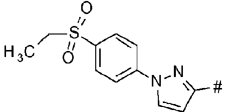 |
| R3-5 | 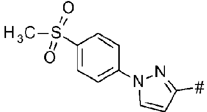 | | R3-10 | 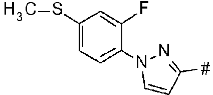 |
| | | | R3-11 | 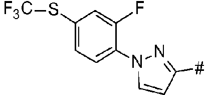 |
| R3-12 | 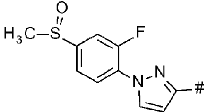 | | R3-21 | 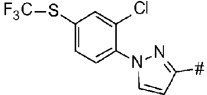 |
| R3-13 | 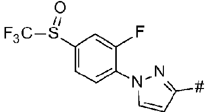 | | R3-22 | 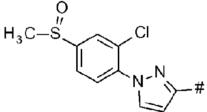 |
| R3-14 | 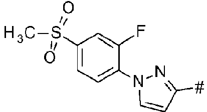 | | R3-23 | 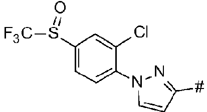 |
| R3-15 | 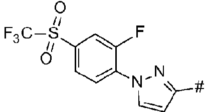 | | R3-24 | 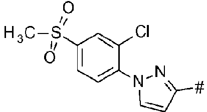 |
| R3-16 | 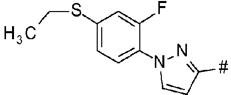 | | R3-25 | 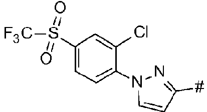 |
| R3-17 | 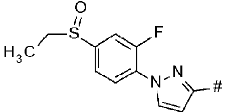 | | R3-26 | 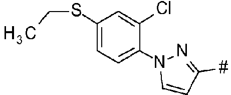 |
| R3-18 | 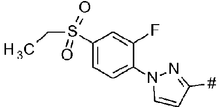 | | R3-27 | 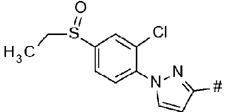 |
| R3-19 | 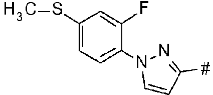 | | R3-28 | 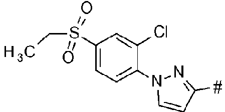 |
| R3-20 | 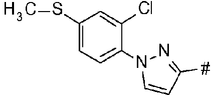 | | R3-29 | 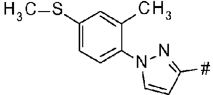 |
| R3-30 | 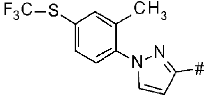 | | R3-39 | 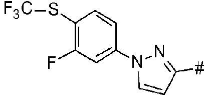 |
| R3-31 | 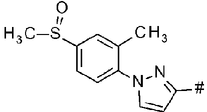 | | R3-40 | 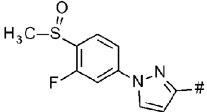 |
| R3-32 | 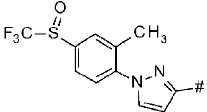 | | R3-41 | 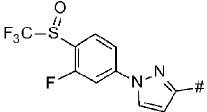 |
| R3-33 | 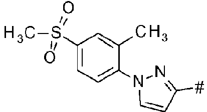 | | R3-42 | 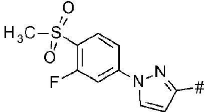 |
| R3-34 | 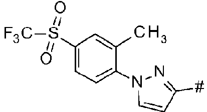 | | R3-43 | 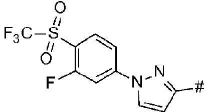 |
| R3-35 | 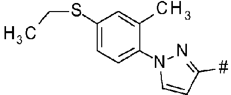 | | R3-44 | 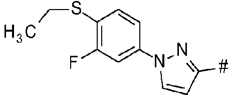 |
| R3-36 | 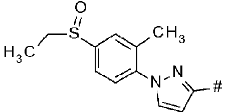 | | R3-45 | 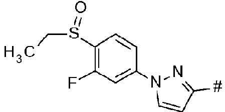 |
| R3-37 | 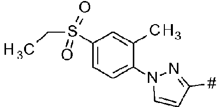 | | R3-46 | 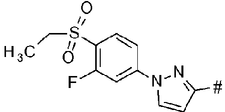 |
| R3-38 | 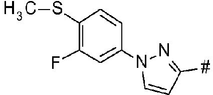 | | R3-47 | 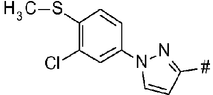 |
| R3-48 | 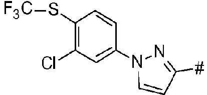 | | R3-57 | 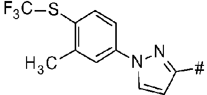 |
| R3-49 | 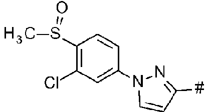 | | R3-58 | 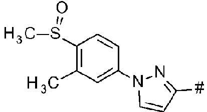 |
| R3-50 | 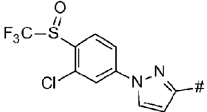 | | R3-59 | 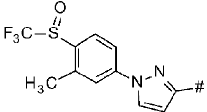 |
| R3-51 | 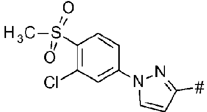 | | R3-60 | 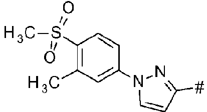 |
| R3-52 | 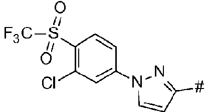 | | R3-61 | 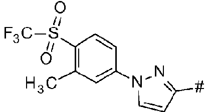 |
| R3-53 | 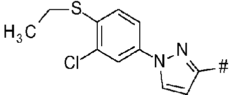 | | R3-62 | 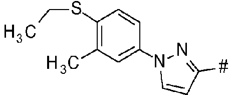 |
| R3-54 | 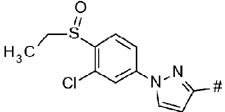 | | R3-63 | 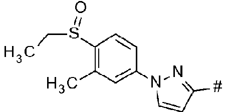 |
| R3-55 | 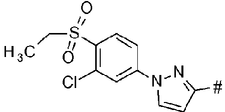 | | R3-64 | 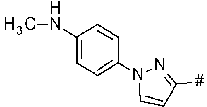 |
| R3-56 | 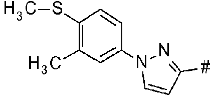 | | R3-65 | 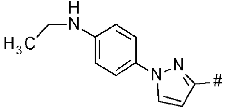 |
| R3-66 | 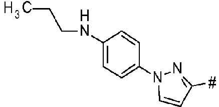 | | R3-76 | 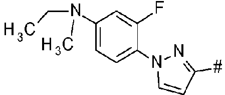 |
| R3-67 | 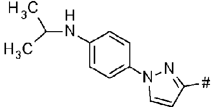 | | R3-77 | 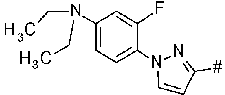 |
| R3-68 | 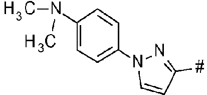 | | R3-78 | 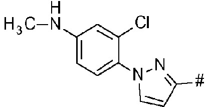 |
| R3-69 | 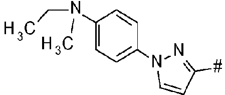 | | R3-79 | 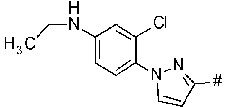 |
| R3-70 | 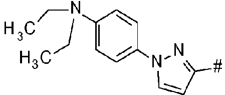 | | R3-80 | 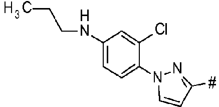 |
| R3-71 | 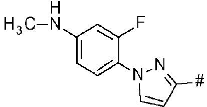 | | R3-81 | 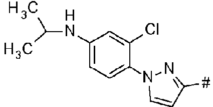 |
| R3-72 | 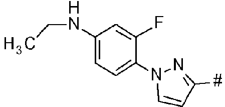 | | R3-82 | 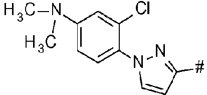 |
| R3-73 | 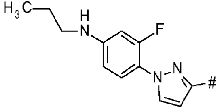 | | R3-83 | 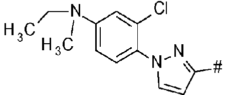 |
| R3-74 | 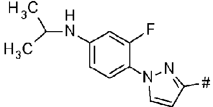 | | R3-84 | 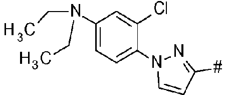 |
| R3-75 | 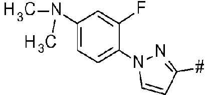 | | R3-85 | 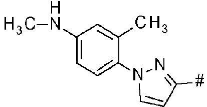 |
| R3-86 | 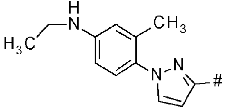 | | R3-95 | 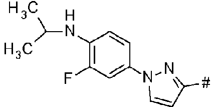 |
| R3-87 | 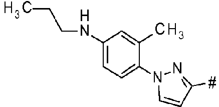 | | R3-96 | 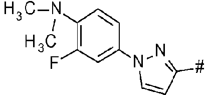 |
| R3-88 | 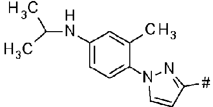 | | R3-97 | 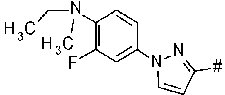 |
| R3-89 | 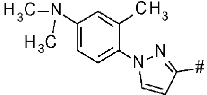 | | R3-98 | 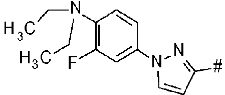 |
| R3-90 | 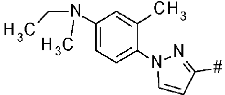 | | R3-99 | 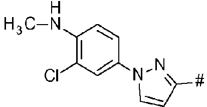 |
| R3-91 | 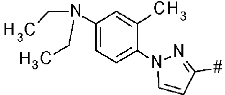 | | R3-100 | 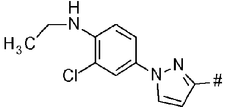 |
| R3-92 | 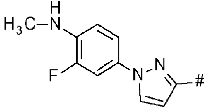 | | R3-101 | 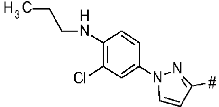 |
| R3-93 | 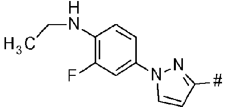 | | R3-102 | 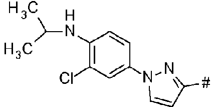 |
| R3-94 | 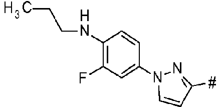 | | R3-103 | 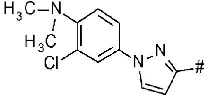 |
| | | | R3-104 | 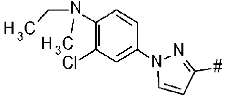 |
| R3-105 | 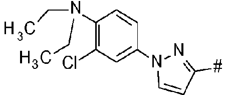 | | R3-114 | 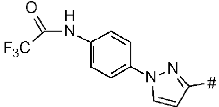 |
| R3-106 | 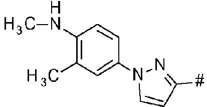 | | R3-115 | 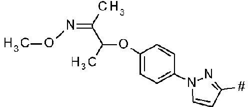 |
| R3-107 | 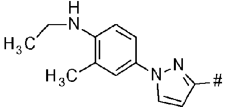 | | R3-116 | 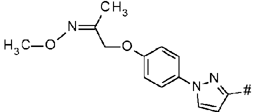 |
| R3-108 | 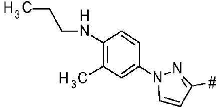 | | R3-117 | 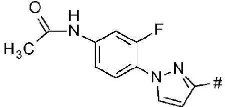 |
| R3-109 | 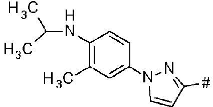 | | R3-118 | 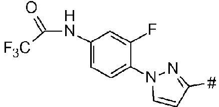 |
| R3-110 | 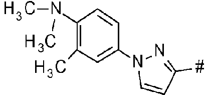 | | | |
| R3-111 | 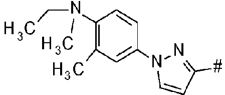 | | R3-119 | 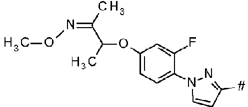 |
| R3-112 | 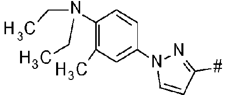 | | R3-120 | 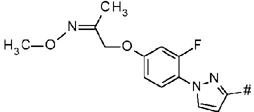 |
| R3-113 | 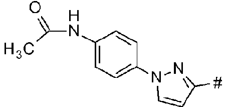 | | R3-121 | 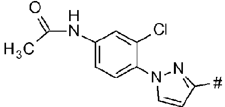 |
| | | | R3-122 | 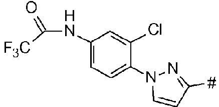 |
| R3-123 | 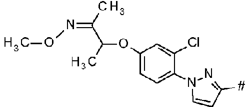 | | R3-132 | 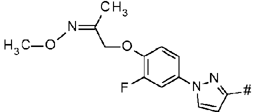 |
| R3-124 | 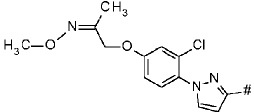 | | R3-133 | 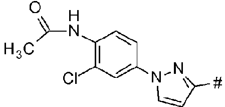 |
| R3-125 | 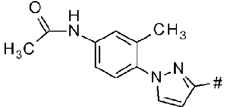 | | R3-134 | 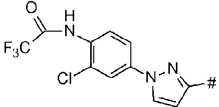 |
| R3-126 | 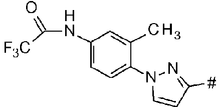 | | R3-135 | 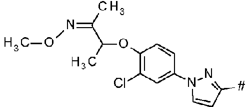 |
| R3-127 | 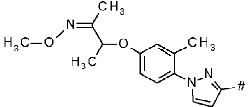 | | R3-136 | 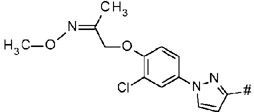 |
| R3-128 | 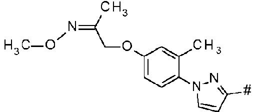 | | R3-137 | 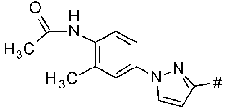 |
| R3-129 | 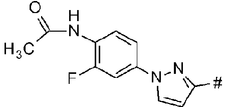 | | R3-138 | 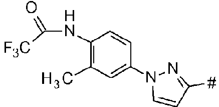 |
| R3-130 | 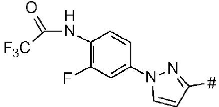 | | R3-139 | 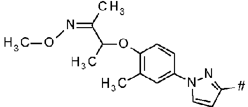 |
| R3-131 | 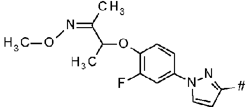 | | R3-140 | 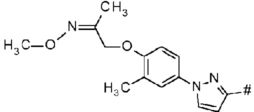 |

Preferred embodiments of the invention relate to compounds I, wherein the group R³ is R3-A, in particular R3-2 [1-(4-trifluoromethylsulfanylphenyl)-pyrazol-3-yl].
Table 1: Compounds of formula I, wherein R¹ and R² are defined as in line R-1 of table R, R⁴ is defined as in line R4-10 of table R4, Y is defined as in line Y-1 of table Y, and the meaning for the moiety R^{C}-R^{B}-R³ for each compound corresponds to one line of table A.
Table 2: Compounds of formula I, wherein R¹ and R² are defined as in line R-1 of table R, R⁴ is defined as in line R4-11 of table R4, Y is defined as in line Y-1 of table Y, and the meaning for the moiety R^{C}-R^{B}-R³ for each compound corresponds to one line of table A.
Table 3: Compounds of formula I, wherein R¹ and R² are defined as in line R-1 of table R, R⁴ is defined as in line R4-12 of table R4, Y is defined as in line Y-1 of table Y, and the meaning for the moiety R^{C}-R^{B}-R³ for each compound corresponds to one line of table A.
Table 4: Compounds of formula I, wherein R¹ and R² are defined as in line R-1 of table R, R⁴ is defined as in line R4-13 of table R4, Y is defined as in line Y-1 of table Y, and the meaning for the moiety R^{C}-R^{B}-R³ for each compound corresponds to one line of table A.
Table 5: Compounds of formula I, wherein R¹ and R² are defined as in line R-1 of table R, R⁴ is defined as in line R4-14 of table R4, Y is defined as in line Y-1 of table Y, and the meaning for the moiety R^{C}-R^{B}-R³ for each compound corresponds to one line of table A.
Table 6: Compounds of formula I, wherein R¹ and R² are defined as in line R-1 of table R, R⁴ is defined as in line R4-15 of table R4, Y is defined as in line Y-1 of table Y, and the meaning for the moiety R^{C}-R^{B}-R³ for each compound corresponds to one line of table A.
Table 7: Compounds of formula I, wherein R¹ and R² are defined as in line R-1 of table R, R⁴ is defined as in line R4-16 of table R4, Y is defined as in line Y-1 of table Y, and the meaning for the moiety R^{C}-R^{B}-R³ for each compound corresponds to one line of table A.
Table 8: Compounds of formula I, wherein R¹ and R² are defined as in line R-1 of table R, R⁴ is defined as in line R4-17 of table R4, Y is defined as in line Y-1 of table Y, and the meaning for the moiety R^{C}-R^{B}-R³ for each compound corresponds to one line of table A.
Table 9: Compounds of formula I, wherein R¹ and R² are defined as in line R-1 of table R, R⁴ is defined as in line R4-18 of table R4, Y is defined as in line Y-1 of table Y, and the meaning for the moiety R^{C}-R^{B}-R³ for each compound corresponds to one line of table A.
Table 10: Compounds of formula I, wherein R¹ and R² are defined as in line R-1 of table R, R⁴ is defined as in line R4-19 of table R4, Y is defined as in line Y-1 of table Y, and the meaning for the moiety R^{C}-R^{B}-R³ for each compound corresponds to one line of table A.
Table 11: Compounds of formula I, wherein R¹ and R² are defined as in line R-1 of table R, R⁴ is defined as in line R4-20 of table R4, Y is defined as in line Y-1 of table Y, and the meaning for the moiety R^{C}-R^{B}-R³ for each compound corresponds to one line of table A.
Table 12: Compounds of formula I, wherein R¹ and R² are defined as in line R-1 of table R, R⁴ is defined as in line R4-21 of table R4, Y is defined as in line Y-1 of table Y, and the meaning for the moiety R^{C}-R^{B}-R³ for each compound corresponds to one line of table A.
Table 13: Compounds of formula I, wherein R¹ and R² are defined as in line R-1 of table R, R⁴ is defined as in line R4-22 of table R4, Y is defined as in line Y-1 of table Y, and the meaning for the moiety R^{C}-R^{B}-R³ for each compound corresponds to one line of table A.
Table 14: Compounds of formula I, wherein R¹ and R² are defined as in line R-1 of table R, R⁴ is defined as in line R4-23 of table R4, Y is defined as in line Y-1 of table Y, and the meaning for the moiety R^{C}-R^{B}-R³ for each compound corresponds to one line of table A.
Table 15: Compounds of formula I, wherein R¹ and R² are defined as in line R-1 of table R, R⁴ is defined as in line R4-24 of table R4, Y is defined as in line Y-1 of table Y, and the meaning for the moiety R^{C}-R^{B}-R³ for each compound corresponds to one line of table A.
Table 16: Compounds of formula I, wherein R¹ and R² are defined as in line R-1 of table R, R⁴ is defined as in line R4-25 of table R4, Y is defined as in line Y-1 of table Y, and the meaning for the moiety R^{C}-R^{B}-R³ for each compound corresponds to one line of table A.
Table 17: Compounds of formula I, wherein R¹ and R² are defined as in line R-1 of table R, R⁴ is defined as in line R4-26 of table R4, Y is defined as in line Y-1 of table Y, and the meaning for the moiety R^{C}-R^{B}-R³ for each compound corresponds to one line of table A.
Table 18: Compounds of formula I, wherein R¹ and R² are defined as in line R-1 of table R, R⁴ is defined as in line R4-27 of table R4, Y is defined as in line Y-1 of table Y, and the meaning for the moiety R^{C}-R^{B}-R³ for each compound corresponds to one line of table A.
Table 19: Compounds of formula I, wherein R¹ and R² are defined as in line R-1 of table R, R⁴ is defined as in line R4-28 of table R4, Y is defined as in line Y-1 of table Y, and the meaning for the moiety R^{C}-R^{B}-R³ for each compound corresponds to one line of table A.
Table 20: Compounds of formula I, wherein R¹ and R² are defined as in line R-1 of table R, R⁴ is defined as in line R4-29 of table R4, Y is defined as in line Y-1 of table Y, and the meaning for the moiety R^{C}-R^{B}-R³ for each compound corresponds to one line of table A.
Table 21: Compounds of formula I, wherein R¹ and R² are defined as in line R-1 of table R, R⁴ is defined as in line R4-30 of table R4, Y is defined as in line Y-1 of table Y, and the meaning for the moiety R^{C}-R^{B}-R³ for each compound corresponds to one line of table A.
Table 22: Compounds of formula I, wherein R¹ and R² are defined as in line R-1 of table R, R⁴ is defined as in line R4-31 of table R4, Y is defined as in line Y-1 of table Y, and the meaning for the moiety R^{C}-R^{B}-R³ for each compound corresponds to one line of table A.
Table 23: Compounds of formula I, wherein R¹ and R² are defined as in line R-1 of table R, R⁴ is defined as in line R4-32 of table R4, Y is defined as in line Y-1 of table Y, and the meaning for the moiety R^{C}-R^{B}-R³ for each compound corresponds to one line of table A.
Table 24: Compounds of formula I, wherein R¹ and R² are defined as in line R-1 of table R, R⁴ is defined as in line R4-33 of table R4, Y is defined as in line Y-1 of table Y, and the meaning for the moiety R^{C}-R^{B}-R³ for each compound corresponds to one line of table A.
Table 25: Compounds of formula I, wherein R¹ and R² are defined as in line R-1 of table R, R⁴ is defined as in line R4-34 of table R4, Y is defined as in line Y-1 of table Y, and the meaning for the moiety R^{C}-R^{B}-R³ for each compound corresponds to one line of table A.
Table 26: Compounds of formula I, wherein R¹ and R² are defined as in line R-1 of table R, R⁴ is defined as in line R4-35 of table R4, Y is defined as in line Y-1 of table Y, and the meaning for the moiety R^{C}-R^{B}-R³ for each compound corresponds to one line of table A.
Table 27: Compounds of formula I, wherein R¹ and R² are defined as in line R-1 of table R, R⁴ is defined as in line R4-36 of table R4, Y is defined as in line Y-1 of table Y, and the meaning for the moiety R^{C}-R^{B}-R³ for each compound corresponds to one line of table A.
Table 28: Compounds of formula I, wherein R¹ and R² are defined as in line R-1 of table R, R⁴ is defined as in line R4-37 of table R4, Y is defined as in line Y-1 of table Y, and the meaning for the moiety R^{C}-R^{B}-R³ for each compound corresponds to one line of table A.
Table 29: Compounds of formula I, wherein R¹ and R² are defined as in line R-1 of table R, R⁴ is defined as in line R4-38 of table R4, Y is defined as in line Y-1 of table Y, and the meaning for the moiety R^{C}-R^{B}-R³ for each compound corresponds to one line of table A.
Table 30: Compounds of formula I, wherein R¹ and R² are defined as in line R-1 of table R, R⁴ is defined as in line R4-39 of table R4, Y is defined as in line Y-1 of table Y, and the meaning for the moiety R^{C}-R^{B}-R³ for each compound corresponds to one line of table A.
Table 31: Compounds of formula I, wherein R¹ and R² are defined as in line R-1 of table R, R⁴ is defined as in line R4-40 of table R4, Y is defined as in line Y-1 of table Y, and the meaning for the moiety R^{C}-R^{B}-R³ for each compound corresponds to one line of table A.
Table 32: Compounds of formula I, wherein R¹ and R² are defined as in line R-1 of table R, R⁴ is defined as in line R4-41 of table R4, Y is defined as in line Y-1 of table Y, and the meaning for the moiety R^{C}-R^{B}-R³ for each compound corresponds to one line of table A.
Table 33: Compounds of formula I, wherein R¹ and R² are defined as in line R-1 of table R, R⁴ is defined as in line R4-42 of table R4, Y is defined as in line Y-1 of table Y, and the meaning for the moiety R^{C}-R^{B}-R³ for each compound corresponds to one line of table A.
Table 34: Compounds of formula I, wherein R¹ and R² are defined as in line R-1 of table R, R⁴ is defined as in line R4-43 of table R4, Y is defined as in line Y-1 of table Y, and the meaning for the moiety R^{C}-R^{B}-R³ for each compound corresponds to one line of table A.
Table 35: Compounds of formula I, wherein R¹ and R² are defined as in line R-1 of table R, R⁴ is defined as in line R4-44 of table R4, Y is defined as in line Y-1 of table Y, and the meaning for the moiety R^{C}-R^{B}-R³ for each compound corresponds to one line of table A.
Table 36: Compounds of formula I, wherein R¹ and R² are defined as in line R-1 of table R, R⁴ is defined as in line R4-45 of table R4, Y is defined as in line Y-1 of table Y, and the meaning for the moiety R^{C}-R^{B}-R³ for each compound corresponds to one line of table A.
Tables 37 to 72: Compounds of formula I, wherein R¹ and R² are defined as line R-1 of Table R, R⁴ is defined as in Tables 1 to 36, Y is defined as in line Y-2 instead of Y-1 of Table Y, and the meaning for the moiety R^{C}-R^{B}-R³ for each compound corresponds to one line of Table A.
Tables 73 to 108: Compounds of formula I, wherein R¹ and R² are defined as line R-1 of Table R, R⁴ is defined as in Tables 1 to 36, Y is defined as in line Y-3 instead of Y-1 of Table Y, and the meaning for the moiety R^{C}-R^{B}-R³ for each compound corresponds to one line of Table A.
Tables 109 to 216: Compounds of formula I, wherein R⁴ and Y are defined as in Tables 1 to 108, R¹ and R² are defined as in line R-2 instead of R-1 of Table R and the meaning for the moiety R^{C}-R^{B}-R³ for each compound corresponds to one line of Table A.
Tables 217 to 324: Compounds of formula I, wherein R⁴ and Y are defined as in Tables 1 to 108, R¹ and R² are defined as in line R-3 instead of R-1 of Table R and the meaning for the moiety R^{C}-R^{B}-R³ for each compound corresponds to one line of Table A.
Tables 325 to 432: Compounds of formula I, wherein R⁴ and Y are defined as in Tables 1 to 108, R¹ and R² are defined as in line R-4 instead of R-1 of Table R and the meaning for the moiety R^{C}-R^{B}-R³ for each compound corresponds to one line of Table A.
Tables 433 to 540: Compounds of formula I, wherein R⁴ and Y are defined as in Tables 1 to 108, R¹ and R² are defined as in line R-5 instead of R-1 of Table R and the meaning for the moiety R^{C}-R^{B}-R³ for each compound corresponds to one line of Table A.
Tables 541 to 648: Compounds of formula I, wherein R⁴ and Y are defined as in Tables 1 to 108, R¹ and R² are defined as in line R-6 instead of R-1 of Table R and the meaning for the moiety R^{C}-R^{B}-R³ for each compound corresponds to one line of Table A.
Tables 649 to 756: Compounds of formula I, wherein R⁴ and Y are defined as in Tables 1 to 108, R¹ and R² are defined as in line R-7 instead of R-1 of Table R and the meaning for the moiety R^{C}-R^{B}-R³ for each compound corresponds to one line of Table A.
Tables 757 to 864: Compounds of formula I, wherein R⁴ and Y are defined as in Tables 1 to 108, R¹ and R² are defined as in line R-8 instead of R-1 of Table R and the meaning for the moiety R^{C}-R^{B}-R³ for each compound corresponds to one line of Table A.
Tables 865 to 972: Compounds of formula I, wherein R⁴ and Y are defined as in Tables 1 to 108, R¹ and R² are defined as in line R-9 instead of R-1 of Table R and the meaning for the moiety R^{C}-R^{B}-R³ for each compound corresponds to one line of Table A.
Tables 973 to 1080: Compounds of formula I, wherein R⁴ and Y are defined as in Tables 1 to 360, R¹ and R² are defined as in line R-10 instead of R-1 of Table R and the meaning for the moiety R^{C}-R^{B}-R³ for each compound corresponds to one line of Table A.
Tables 1081 to 1188: Compounds of formula I, wherein R⁴ and Y are defined as in Tables 1 to 108, R¹ and R² are defined as in line R-11 instead of R-1 of Table R and the meaning for the moiety R^{C}-R^{B}-R³ for each compound corresponds to one line of Table A.
Tables 1189 to 1296: Compounds of formula I, wherein R⁴ and Y are defined as in Tables 1 to 108, R¹ and R² are defined as in line R-12 instead of R-1 of Table R and the meaning for the moiety R^{C}-R^{B}-R³ for each compound corresponds to one line of Table A.
Tables 1297 to 1404: Compounds of formula I, wherein R⁴ and Y are defined as in Tables 1 to 108, R¹ and R² are defined as in line R-13 instead of R-1 of Table R and the meaning for the moiety R^{C}-R^{B}-R³ for each compound corresponds to one line of Table A.
Tables 1405 to 1512: Compounds of formula I, wherein R⁴ and Y are defined as in Tables 1 to 108, R¹ and R² are defined as in line R-14 instead of R-1 of Table R and the meaning for the moiety R^{C}-R^{B}-R³ for each compound corresponds to one line of Table A.
Tables 1513 to 1620: Compounds of formula I, wherein R⁴ and Y are defined as in Tables 1 to 108, R¹ and R² are defined as in line R-15 instead of R-1 of Table R and the meaning for the moiety R^{C}-R^{B}-R³ for each compound corresponds to one line of Table A.
Tables 1621 to 1728: Compounds of formula I, wherein R⁴ and Y are defined as in Tables 1 to 108, R¹ and R² are defined as in line R-16 instead of R-1 of Table R and the meaning for the moiety R^{C}-R^{B}-R³ for each compound corresponds to one line of Table A.
Tables 1729 to 1836: Compounds of formula I, wherein R⁴ and Y are defined as in Tables 1 to 108, R¹ and R² are defined as in line R-17 instead of R-1 of Table R and the meaning for the moiety R^{C}-R^{B}-R³ for each compound corresponds to one line of Table A.
Tables 1837 to 1944: Compounds of formula I, wherein R⁴ and Y are defined as in Tables 1 to 108, R¹ and R² are defined as in line R-18 instead of R-1 of Table R and the meaning for the moiety R^{C}-R^{B}-R³ for each compound corresponds to one line of Table A.
Tables 1945 to 2052: Compounds of formula I, wherein R⁴ and Y are defined as in Tables 1 to 108, R¹ and R² are defined as in line R-19 instead of R-1 of Table R and the meaning for the moiety R^{C}-R^{B}-R³ for each compound corresponds to one line of Table A.
Tables 2053 to 2160: Compounds of formula I, wherein R⁴ and Y are defined as in Tables 1 to 108, R¹ and R² are defined as in line R-20 instead of R-1 of Table R and the meaning for the moiety R^{C}-R^{B}-R³ for each compound corresponds to one line of Table A.
Tables 2161 to 2268: Compounds of formula I, wherein R⁴ and Y are defined as in Tables 1 to 108, R¹ and R² are defined as in line R-21 instead of R-1 of Table R and the meaning for the moiety R^{C}-R^{B}-R³ for each compound corresponds to one line of Table A.
Tables 2269 to 2376: Compounds of formula I, wherein R⁴ and Y are defined as in Tables 1 to 108, R¹ and R² are defined as in line R-22 instead of R-1 of Table R and the meaning for the moiety R^{C}-R^{B}-R³ for each compound corresponds to one line of Table A.
Tables 2377 to 2484: Compounds of formula I, wherein R⁴ and Y are defined as in Tables 1 to 108, R¹ and R² are defined as in line R-23 instead of R-1 of Table R and the meaning for the moiety R^{C}-R^{B}-R³ for each compound corresponds to one line of Table A.
Tables 2485 to 2592: Compounds of formula I, wherein R⁴ and Y are defined as in Tables 1 to 108, R¹ and R² are defined as in line R-24 instead of R-1 of Table R and the meaning for the moiety R^{C}-R^{B}-R³ for each compound corresponds to one line of Table A.
Tables 2593 to 2700: Compounds of formula I, wherein R⁴ and Y are defined as in Tables 1 to 360, R¹ and R² are defined as in line R-25 instead of R-1 of Table R and the meaning for the moiety R^{C}-R^{B}-R³ for each compound corresponds to one line of Table A.
Tables 2701 to 2808: Compounds of formula I, wherein R⁴ and Y are defined as in Tables 1 to 108, R¹ and R² are defined as in line R-26 instead of R-1 of Table R and the meaning for the moiety R^{C}-R^{B}-R³ for each compound corresponds to one line of Table A.
Tables 2809 to 2916: Compounds of formula I, wherein R⁴ and Y are defined as in Tables 1 to 108, R¹ and R² are defined as in line R-27 instead of R-1 of Table R and the meaning for the moiety R^{C}-R^{B}-R³ for each compound corresponds to one line of Table A.
Tables 2917 to 3024: Compounds of formula I, wherein R⁴ and Y are defined as in Tables 1 to 108, R¹ and R² are defined as in line R-28 instead of R-1 of Table R and the meaning for the moiety R^{C}-R^{B}-R³ for each compound corresponds to one line of Table A.
Tables 3025 to 3132: Compounds of formula I, wherein R⁴ and Y are defined as in Tables 1 to 108, R¹ and R² are defined as in line R-29 instead of R-1 of Table R and the meaning for the moiety R^{C}-R^{B}-R³ for each compound corresponds to one line of Table A.
Tables 3133 to 3240: Compounds of formula I, wherein R⁴ and Y are defined as in Tables 1 to 108, R¹ and R² are defined as in line R-30 instead of R-1 of Table R and the meaning for the moiety R^{C}-R^{B}-R³ for each compound corresponds to one line of Table A.
Tables 3241 to 3348: Compounds of formula I, wherein R⁴ and Y are defined as in Tables 1 to 108, R¹ and R² are defined as in line R-31 instead of R-1 of Table R and the meaning for the moiety R^{C}-R^{B}-R³ for each compound corresponds to one line of Table A.
Tables 3349 to 3456: Compounds of formula I, wherein R⁴ and Y are defined as in Tables 1 to 108, R¹ and R² are defined as in line R-32 instead of R-1 of Table R and the meaning for the moiety R^{C}-R^{B}-R³ for each compound corresponds to one line of Table A.
Tables 3457 to 3564: Compounds of formula I, wherein R⁴ and Y are defined as in Tables 1 to 108, R¹ and R² are defined as in line R-33 instead of R-1 of Table R and the meaning for the moiety R^{C}-R^{B}-R³ for each compound corresponds to one line of Table A.
Tables 3565 to 3672: Compounds of formula I, wherein R⁴ and Y are defined as in Tables 1 to 108, R¹ and R² are defined as in line R-34 instead of R-1 of Table R and the meaning for the moiety R^{C}-R^{B}-R³ for each compound corresponds to one line of Table A.
Tables 3673 to 3780: Compounds of formula I, wherein R⁴ and Y are defined as in Tables 1 to 108, R¹ and R² are defined as in line R-35 instead of R-1 of Table R and the meaning for the moiety R^{C}-R^{B}-R³ for each compound corresponds to one line of Table A.
Tables 3781 to 3888: Compounds of formula I, wherein R⁴ and Y are defined as in Tables 1 to 108, R¹ and R² are defined as in line R-36 instead of R-1 of Table R and the meaning for the moiety R^{C}-R^{B}-R³ for each compound corresponds to one line of Table A.

Preference is also given to the uses, methods, mixtures and compositions, wherein the definitions (such as phytopathogenic fungi, treatments, crops, compounds II, further active ingredients, solvents, solid carriers) have independently of each other or more preferably in combination the following meanings and even more preferably in combination (any possible combination of 2 or more definitions as provided herein) with the preferred meanings of compounds I herein:

According to one embodiment of the invention, the invention also relates to a method for combating phytopathogenic fungi containing a mutation in the mitochondrial cytochrome b gene conferring resistance to Qo inhibitors, comprising: treating the phytopathogenic fungi containing a mutation in the mitochondrial cytochrome b gene conferring resistance to Qo inhibitors or the materials, plants, the soil or seeds that are at risk of being diseased from phytopathogenic fungi containing a mutation in the mitochondrial cytochrome b gene conferring resistance to Qo inhibitors with an effective amount of at least one compound I, or a composition comprising it thereof.

The term "phytopathogenic fungi containing a mutation in the mitochondrial cytochrome b gene conferring resistance to Qo inhibitors" ist be understood that at least 10% of the fungal isolates to be controlled contain a mutation in the mitochondrial cytochrome b gene conferring resistance to Qo inhibitors, more preferably at least 30%, even more preferably at least 50%, and most preferably at least 75% of the fungi, in particular between 90 and 100%.

It has been observed under field conditions that populations of phytopathogenic fungi apparently consisting of non-resistant strains can readily develop resistance. The compounds can be applied under such conditions, too, in order to prevent the formation of resistance and the spread of resistant strains altogether. In this regard it is useful that they have strong activity against non-resistant phytopathogenic fungi also.

According to another embodiment, the method for combating phytopathogenic fungi, comprises: a) identifying the phytopathogenic fungi containing a mutation in the mitochondrial cytochrome b gene conferring resistance to Qo inhibitors, or the materials, plants, the soil or seeds that are at risk of being diseased from phytopathogenic fungi as defined herein, and b) treating said fungi or the materials, plants, the soil or seeds with an effective amount of at least one compound I, or a composition comprising it thereof.

According to another embodiment of the invention, the invention also relates to a method for combating phytopathogenic fungi containing a mutation in the mitochondrial cytochrome b gene conferring resistance to Qo inhibitors, comprising: treating the phytopathogenic fungi whereof at least 10% contain a mutation in the mitochondrial cytochrome b gene conferring resistance to Qo inhibitors or the materials, plants, the soil or seeds that are at risk of being diseased from phytopathogenic fungi containing a mutation in the mitochondrial cytochrome b gene conferring resistance to Qo inhibitors with an effective amount of at least one compound I, or a composition comprising it thereof; more preferably at least 30%, even more preferably at least 50%, and most preferably at least 75% of the fungi contain a mutation in the mitochondrial cytochrome b gene conferring resistance to Qo inhibitors.

According to one embodiment of the use and the method for combating phytopathogenic fungi, wherein the mutation in the mitochondrial cytochrome b gene of the phytopathogenic fungi is G143A.

According to another embodiment, the phytopathogenic fungi are selected from the group consisting of basidomycetes, ascomycetes, and oomycetes.

According to a further embodiment, the phytopathogenic fungi are selected from the group consisting of *Alternaria alternata, Blumeria graminis, Pyriculania oryzae* (also known *as Magnaporthe grisea), Septoria tritici* (also known as *Mycosphaerella graminicola), Mycosphaerella fijiensis, Venturia inaequalis, Pyrenophora teres, Pyrenophona tritici-repentis* and *Plasmopara viticola,* in particular *Septoria tritici.*

The compounds I and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans. Preferably, treatment of plant propagation materials with compounds I is peformed by coating.

Consequently, the invention also relates to plant propagation material compising compounds I as defined herein.

The term "cultivated plants" is to be understood as including plants which have been modified by breeding, mutagenesis or genetic engineering including but not limiting to agricultural biotech products on the market or in development (cf. http://ceragmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted posttranslational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. *or Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum)* or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and compositions thereof, respectively, are particularly suitable for controlling the following plant diseases:
*Albugo* spp. (white rust) on ornamentals, vegetables (e. g. ***A.** candida)* and sunflowers (e. g. ***A.** tragopogonis*); *Alternaria* spp. (Alternaria leaf spot) on vegetables, rape (*A*. *brassicola* or *brassicae*), sugar beets (*A*. *tenuis),* fruits, rice, soybeans, potatoes (e. g. *A. solani* or ***A.** alternata*), tomatoes (e. g. ***A.** solani* or ***A.** alternata*) and wheat; *Aphano-myces* spp. on sugar beets and vegetables; *Ascochyta* spp. on cereals and vegetables, e. g. ***A.** tritici* (anthracnose) on wheat and ***A.** hordei* on barley; *Bipolaris* and *Drechslera* spp. (teleomorph: *Cochliobolus* spp.), e. g. Southern leaf blight *(D. maydis)* or Northern leaf blight *(B. zeicola*) on corn, e. g. spot blotch *(B. sorokiniana)* on cereals and e.g. *B*. *oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma)* spp. (rot or wilt) on broadleaved trees and evergreens, e. g. *C. ulmi* (Dutch elm disease) on elms; *Cercospora* spp. (Cercospora leaf spots) on corn (e.g. Gray leaf spot: *C. zeae-maydis),* rice, sugar beets (e. g. *C. beticola),* sugar cane, vegetables, coffee, soybeans (e. g. *C. sojina* or ***C.** kikuchii*) and rice; *Cladosporium* spp. on tomatoes (e. g. *C. fulvum:* leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C*. *carbonum*), cereals (e. g. *C. sativus,* anamorph: *B. sorokiniana*) and rice (e. g. ***C.** miyabeanus,* anamorph: *H. oryzae*); *Colletotrichum* (teleomorph: *Glomerella)* spp. (anthracnose) on cotton (e. g. *C. gossypii*), corn (e. g. *C. graminicola:* Anthracnose stalk rot), soft fruits, potatoes (e. g. *C. coccodes:* black dot), beans (e. g. *C. lindemuthianum*) and soybeans (e. g. *C. truncatum* or ***C.** gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri*: Black Foot Disease) and ornamentals; *Dematophora* (teleomorph: *Rosellinia)* necatrix (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D. tritici-repentis:* tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa*; *Elsinoe* spp. on pome fruits *(E. pyri*), soft fruits *(E. veneta:* anthracnose) and vines (*E. ampelina:* anthracnose); *Entyloma oryzae* (leaf smut) on rice; *Epicoccum* spp. (black mold) on wheat; *Erysiphe* spp. (powdery mildew) on sugar beets *(E. betae*), vegetables (e. g. *E. pisi*), such as cucurbits (e. g. *E. cichoracearum),* cabbages, rape (e. g. *E. cruciferarum); Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella)* spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi:* Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium) nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or ***M.** fijiensis* (black Sigatoka disease) on bananas; *Peronospora* spp. (downy mildew) on cabbage (e. g. P. *brassicae*), rape (e. g. *P. parasitica),* onions (e. g. *P. destructor*), tobacco *(P. tabacina*) and soybeans (e. g. *P. manshurica); Phakopsora pachyrhizi* and *P. meibomiae* (soybean rust) on soybeans; *Phialophora* spp. e. g. on vines (e. g. *P. tracheiphila* and ***P.** tetraspora*) and soybeans (e. g. *P. gregata:* stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P. phaseoli,* teleomorph: *Diaporthe phaseolorum); Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*), potatoes and tomatoes (e. g. *P. infestans:* late blight) and broad-leaved trees (e. g. *P. ramorum:* sudden oak death); *Plasmodiophora brassicae* (club root) on cabbage, rape, radish and other plants; *Plasmopara* spp., e. g. ***P.** viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P*. *graminis)* and sugar beets *(P. betae)* and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. ***P.** cubensis* on cucurbits or ***P.** humili* on hop; *Pseudopezicula tracheiphila* (red fire disease or ,rotbrenner', anamorph: *Phialophora)* on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or ***P.** recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or ***P.** teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or ***P.** aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or ***R.** cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and ***S.** attenuatum* (sheath rot) on rice; *Sclerotinia* spp. (stem rot or white mold) on vegetables and field crops, such as rape, sunflowers (e. g. ***S.** sclerotiorum)* and soybeans (e. g. ***S.** rolfsii* or ***S.** sclerotiorum*); *Septoria* spp. on various plants, e. g. *S. glycines* (brown spot) on soybeans, ***S.** tritici* (Septoria blotch) on wheat and ***S.*** (syn. *Stagonospora)* nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. ***S.** turcicum,* syn. *Helminthosporium turcicum*) and turf; *Sphacelotheca* spp. (smut) on corn, (e. g. ***S.** reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. ***S.** nodorum* (Stagonospora blotch, teleomorph: *Leptosphaeria* [syn. *Phaeosphaeria] nodorum)* on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis:* corn smut) and sugar cane; *Venturia* spp. (scab) on apples (e. g. V. *inaequalis)* and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucorspp.,* and in addition in the protection of stored products and harvest the following yeast fungi are worthy of note: *Candida* spp. and *Saccharomyces cerevisae.*

The compounds I and compositions thereof, resepectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinyl-alcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound I and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a compound I and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a compound I and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e.g. finely divided kaolin) ad 100 wt%.
xii) Granules (GR, FG)
   0.5-30 wt% of a compound I is ground finely and associated with solid carrier (e.g. silicate) ad 100 wt%. Granulation is achieved by extrusion, spray-drying or fluidized bed.
xiii) Ultra-low volume liquids (UL)
   1-50 wt% of a compound I are dissolved in organic solvent (e.g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of active substances, in conjunction with which the compounds I can be used, is intended to illustrate the possible combinations but does not limit them:
A) Respiration inhibitors
   - Inhibitors of complex III at Qₒ site (e.g. strobilurins): azoxystrobin, coumethoxystrobin, coumoxystrobin, dimoxystrobin, enestroburin, fenaminstrobin, fenoxystrobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxymethyl)phenyl)-2-methoxyimino-N-methyl-acetamide, pyribencarb, triclopyricarb/chlorodin-carb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-di-oxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate
   - inhibitors of complex II (e. g. carboxamides): benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1H-pyrazole-4-carboxamide, N-(2-(1,3,3-trimethyl-butyl)-phenyl)-1,3-dimethyl-5-fluoro-1H-pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide, N-(7-fluoro-1,1,3-trimethyl-indan-4-yl)-1,3-dimethyl-pyrazole-4-carboxamide, N-[2-(2,4-dichlorophenyl)-2-methoxy-1-methyl-ethyl]-3-(difluoromethyl)-1-methyl-pyrazole-4-carboxamide;
   - other respiration inhibitors (e.g. complex I, uncouplers): diflumetorim, (5,8-difluoro-quinazolin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutra-zole, penconazole, propiconazole, prothioconazole, simeconazole, tebuconazole, tetraconazole, triadimefon, triadimenol, triticonazole, uniconazole, 1-[*rel*(2*S*;3*R*)-3-(2-chlorophenyl)-2-(2,4-difluorophenyl)-oxiranylmethyl]-5-thio-cyanato-1H-[1,2,4]triazole, 2-[*rel*-(2*S*;3*R*)-3-(2-chlorophenyl)-2-(2,4-difluorophenyl)-oxiranylmethyl]-2H-[1,2,4]triazole-3-thiol, 2-[2-chloro-4-(4-chlorophenoxy)phenyl]-1-(1,2,4-triazol-1-yl)pentan-2-ol, 1-[4-(4-chlorophenoxy)-2-(trifluoromethyl)phenyl]-1-cyclopropyl-2-(1,2,4-triazol-1-yl)ethanol, 2-[4-(4-chlorophenoxy)-2-(trifluorometh-yl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chlorophenoxy)-2-(trifluoromethyl)phenyl]-3-methyl-1-(1,2,4-triazol-1-yl)butan-2-ol, 2-[4-(4-chlorophenoxy)-2-(trifluoromethyl)-phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)-phenyl]-1-(1,2,4-triazol-1-yl)pentan-2-ol, 2-[4-(4-fluorophenoxy)-2-(trifluoromethyl)-phenyl]-1-(1,2,4-triazol-1-yl)propan-2-ol; imidazoles: imazalil, pefurazoate, prochloraz, triflumizol; pyrimidines, pyridines and piperazines: fenarimol, nuarimol, pyrifenox, triforine, [3-(4-chloro-2-fluoro-phenyl)-5-(2,4-difluorophenyl)isoxazol-4-yl]-(3-pyridyl)methanol;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benomyl, carbendazim, fuberidazole, thiabendazole, thiophanate-methyl; triazolopyrimidines: 5-chloro-7-(4-methylpiperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): cyprodinil, mepanipyrim, pyrimethanil;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)ethanesulfonyl)-but-2-yl) carbamic acid-(4-fluorophenyl) ester;
   - compounds affecting cell membrane permeability and fatty acides: propamocarb, propamocarb-hydrochlorid
   - fatty acid amide hydrolase inhibitors: oxathiapiprolin;
H) Inhibitors with Multi Site Action
   - inorganic active substances: Bordeaux mixture, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, hexachlorobenzene, pentachlorphenole and its salts, phthalide, tolylfluanid, N-(4-chloro-2-nitrophenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1 H,5H-[1,4]dithiino[2,3-c:5,6-c']dipyrrole-1,3,5,7(2H,6H)-tetraone;
I) Cell wall synthesis inhibitors
   - inhibitors of glucan synthesis: validamycin, polyoxin B; melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts;
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, tolprocarb, 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl)-1,3-thiazol-2-yl)piperidin-1-yl]ethanone, 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl)-1,3-thiazol-2-yl)piperidin-1 - yl]ethanone, 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl)-1,3-thiazol-2-yl)piperidin-1-yl]ethanone, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, methoxy-acetic acid 6-tert-butyl-8-fluoro-2,3-dimethyl-quinolin-4-yl ester, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole), N-(6-methoxy-pyridin-3-yl) cyclopropanecarboxylic acid amide, 5-chloro-1-(4,6-di-methoxy-pyrimidin-2-yl)-2-methyl-1H-benzoimidazole, 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate, tert-butyl N-[6-[[(Z)-[(1-methyltetrazol-5-yl)-phenyl-methylene]amino]oxymethyl]-2-pyridyl]carbamate, pentyl N-[6-[[(Z)-[(1-methyltetrazol-5-yl)-phenyl-methylene]amino]oxymethyl]-2-pyridyl]carbamate, 2-[2-[(7,8-difluoro-2-methyl-3-quinolyl)oxy]-6-fluoro-phenyl]propan-2-ol, 2-[2-fluoro-6-[(8-fluoro-2-methyl-3-quinolyl)oxy]phenyl]propan-2-ol, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, 3-(4,4,5-trifluoro-3,3-dimethyl-3,4-dihydroisoquinolin-1-yl)quinoline;
L) Antifungal biocontrol agents, plant bioactivators: *Ampelomyces quisqualis* (e.g. AQ 10^{®} from Intrachem Bio GmbH & Co. KG, Germany), *Aspergillus flavus* (e.g. AFLAGUARD^{®} from Syngenta, CH), *Aureobasidium pullulans* (e.g. BOTECTOR^{®} from bio-ferm GmbH, Germany), *Bacillus pumilus* (e.g. NRRL Accession No. B-30087 in SONATA^{®} and BALLAD^{®} Plus from AgraQuest Inc., USA), *Bacillus subtilis* (e.g. isolate NRRL-Nr. B-21661 in RHAPSODY^{®}, SERENADE^{®} MAX and SERENADE^{®} ASO from AgraQuest Inc., USA), *Bacillus subtilis* var. *amyloliquefaciens* FZB24 (e.g. TAEGRO^{®} from Novozyme Biologicals, Inc., USA), *Candida oleophila* I-82 (e.g. ASPIRE^{®} from Ecogen Inc., USA), *Candida saitoana* (e.g. BIOCURE^{®} (in mixture with lysozyme) and BIOCOAT^{®} from Micro Flo Company, USA (BASF SE) and Arysta), Chitosan (e.g. ARMOUR-ZEN from BotriZen Ltd., NZ), *Clonostachys rosea* f. *catenulata,* also named *Gliocladium catenulatum* (e.g. isolate J1446: PRESTOP^{®} from Verdera, Finland), *Coniothyrium minitans* (e.g. CONTANS^{®} from Prophyta, Germany), *Cryphonectria parasitica* (e.g. *Endothia parasitica* from CNICM, France), *Cryptococcus albidus* (e.g. YIELD PLUS^{®} from Anchor Bio-Technologies, South Africa), *Fusarium oxysporum* (e.g. BIOFOX^{®} from S.I.A.P.A., Italy, FUSACLEAN^{®} from Natural Plant Protection, France), *Metschnikowia fructicola* (e.g. SHEMER^{®} from Agrogreen, Israel), *Microdochium dimerum* (e.g. ANTIBOT^{®} from Agrauxine, France), *Phlebiopsis gigantea* (e.g. ROTSOP^{®} from Verdera, Finland), *Pseudozyma flocculosa* (e.g. SPORODEX^{®} from Plant Products Co. Ltd., Canada), *Pythium oligandrum* DV74 (e.g. POLYVERSUM^{®} from Remeslo SSRO, Biopreparaty, Czech Rep.), *Reynoutria sachlinensis* (e.g. REGALIA^{®} from Marrone Biolnnovations, USA), *Talaromyces flavus* V117b (e.g. PROTUS^{®} from Prophyta, Germany), *Trichoderma asperellum* SKT-1 (e.g. ECO-HOPE^{®} from Kumiai Chemical Industry Co., Ltd., Japan), *T. atroviride* LC52 (e.g. SENTINEL^{®} from Agrimm Technologies Ltd, NZ), *T. harzianum* T-22 (e.g. PLANTSHIELD^{®} der Firma BioWorks Inc., USA), *T. harzianum* TH 35 (e.g. ROOT PRO^{®} from Mycontrol Ltd., Israel), *T. harzianum* T-39 (e.g. TRICHODEX^{®} and TRICHODERMA 2000^{®} from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER^{®} WP from Isagro Ricerca, Italy), *T. polysporum* and *T. harzianum* (e.g. BINAB^{®} from BINAB BioInnovation AB, Sweden), *T. stromaticum* (e.g. TRICOVAB^{®} from C.E.P.L.A.C., Brazil), *T. virens* GL-21 (e.g. SOILGARD^{®} from Certis LLC, USA), *T. viride* (e.g. TRIECO^{®} from Ecosense Labs. (India) Pvt. Ltd., Indien, BIO-CURE^{®} F from T. Stanes & Co. Ltd., Indien), *T. virideTV1* (e.g. T. viride TV1 from Agribiotec srl, Italy), *Ulocladium oudemansii* HRU3 (e.g. BOTRY-ZEN^{®} from Botry-Zen Ltd, NZ);
M) Growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron,tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cumyluron, cyprosulfamide, dicamba, difenzoquat, diflufenzopyr, *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester. O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, no-valuron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1 H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - ryanodine receptor inhibitors: chlorantraniliprole, cyantraniliprole, flubendiamide, N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; N-[4-chloro-2-[(di-2-propyl-lambda-4-sulfanyli-dene)carbamoyl]-6-methyl-phenyl]-2-(3-chloro-2-pyridyl)-5-(trifluoromethyl)pyrazole-3-carboxamide; N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; N-[4,6-di-chloro-2-[(diethyl-lambda-4-sulfanylidene)carbamoyl]-phenyl]-2-(3-chloro-2-pyridyl)-5-(difluoromethyl)pyrazole-3-carboxamide; N-[4,6-dibromo-2-[(di-2-propyl-lambda4-sulfanylidene)carbamoyl]-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide; N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide;
   - others: benclothiaz, bifenazate, cartap, flonicamid, pyridalyl, pymetrozine, sulfur, thiocyclam, cyenopyrafen, flupyrazofos, cyflumetofen, amidoflumet, imicyafos, bis-trifluron, pyrifluquinazon and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a, 12b-trimethyl-11-oxo-9-(3-pyridinyl)-2H, 11H-naphtho[2,1-b]pyrano[3,4-e]pyran-3,6-diyl] cyclopropaneacetic acid ester.

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to L), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to L), as described above, is more efficient than combating those fungi with individual compounds I or individual fungicides from groups A) to L). By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

In binary mixtures, i.e. compositions according to the invention comprising one compound I (component 1) and one further active substance (component 2), e. g. one active substance from groups A) to O), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:3 to 3:1.

In ternary mixtures, i.e. compositions according to the invention comprising one compound I (component 1) and a first further active substance (component 2) and a second further active substance (component 3), e. g. two active substances from groups A) to O), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

Preference is also given to mixtures comprising a compound I (component 1) and at least one active substance selected from group A) (component 2) and particularly selected from azoxystrobin, dimoxystrobin, fluoxastrobin, kresoxim-methyl, orysastrobin, picoxystrobin, pyraclostrobin, trifloxystrobin; famoxadone, fenamidone; benzovindiflupyr, bixafen, boscalid, fluopyram, fluxapyroxad, isopyrazam, penflufen, penthiopyrad, sedaxane; ametoctradin, cyazofamid, fluazinam, fentin salts, such as fentin acetate.

Preference is given to mixtures comprising a compound of formula I (component 1) and at least one active substance selected from group B) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, fenarimol, triforine; dodemorph, fenpropimorph, tridemorph, fenpropidin, spiroxamine; fenhexamid.

Preference is given to mixtures comprising a compound of formula I (component 1) and at least one active substance selected from group C) (component 2) and particularly selected from metalaxyl, (metalaxyl-M) mefenoxam, ofurace.

Preference is given to mixtures comprising a compound of formula I (component 1) and at least one active substance selected from group D) (component 2) and particularly selected from benomyl, carbendazim, thiophanate-methyl, ethaboxam, fluopicolide, zoxamide, metrafenone, pyriofenone.

Preference is also given to mixtures comprising a compound I (component 1) and at least one active substance selected from group E) (component 2) and particularly selected from cyprodinil, mepanipyrim, pyrimethanil.

Preference is also given to mixtures comprising a compound I (component 1) and at least one active substance selected from group F) (component 2) and particularly selected from iprodione, fludioxonil, vinclozolin, quinoxyfen.

Preference is also given to mixtures comprising a compound I (component 1) and at least one active substance selected from group G) (component 2) and particularly selected from dimethomorph, flumorph, iprovalicarb, benthiavalicarb, mandipropamid, propamocarb.

Preference is also given to mixtures comprising a compound I (component 1) and at least one active substance selected from group H) (component 2) and particularly selected from copper acetate, copper hydroxide, copper oxychloride, copper sulfate, sulfur, mancozeb, metiram, propineb, thiram, captafol, folpet, chlorothalonil, dichlofluanid, dithianon.

Preference is also given to mixtures comprising a compound I (component 1) and at least one active substance selected from group I) (component 2) and particularly selected from carpropamid and fenoxanil.

Preference is also given to mixtures comprising a compound I (component 1) and at least one active substance selected from group J) (component 2) and particularly selected from acibenzolar-S-methyl, probenazole, tiadinil, fosetyl, fosetyl-aluminium, H₃PO₃ and salts thereof.

Preference is also given to mixtures comprising a compound I (component 1) and at least one active substance selected from group K) (component 2) and particularly selected from cymoxanil, proquinazid and *N*-methyl-2-{1-[(5-methyl-3-trifluoromethyl-1H-pyrazol-1-yl)-acetyl]-piperidin-4-yl}-*N*-[(1R)-1,2,3,4-tetrahydronaphthalen-1-yl]-4-thiazolecarboxamide.

Preference is also given to mixtures comprising a compound I (component 1) and at least one active substance selected from group L) (component 2) and particularly selected from *Bacillus subtilis* strain NRRL No. B-21661, *Bacillus pumilus* strain NRRL No. B-30087 and *Ulocladium oudemansii.*

Accordingly, the present invention furthermore relates to compositions comprising one compound I (component 1) and one further active substance (component 2), which further active substance is selected from the column "Component 2" of the lines B-1 to B-402 of Table B.

A further embodiment relates to the compositions B-1 to B-402 listed in Table B, where a row of Table B corresponds in each case to a fungicidal composition comprising one of the in the present specification individualized compounds of formula I (component 1) and the respective further active substance from groups A) to O) (component 2) stated in the row in question. Preferably, the compositions described comprise the active substances in synergistically effective amounts.

**Table B: Composition comprising one indiviualized compound I and one further active substance from groups A) to O)**

| **Mixture** | **Component 1** | **Component 2** |
|---|---|---|
| B-1 | one individualized compound I | Azoxystrobin |
| B-2 | one individualized compound I | Coumethoxystrobin |
| B-3 | one individualized compound I | Coumoxystrobin |
| B-4 | one individualized compound I | Dimoxystrobin |
| B-5 | one individualized compound I | Enestroburin |
| B-6 | one individualized compound I | Fenaminstrobin |
| B-7 | one individualized compound I | Fenoxystrobin/Flufenoxystrobin |
| B-8 | one individualized compound I | Fluoxastrobin |
| B-9 | one individualized compound I | Kresoxim-methyl |
| B-10 | one individualized compound I | Metominostrobin |
| B-11 | one individualized compound I | Orysastrobin |
| B-12 | one individualized compound I | Picoxystrobin |
| B-13 | one individualized compound I | Pyraclostrobin |
| B-14 | one individualized compound I | Pyrametostrobin |
| B-15 | one individualized compound I | Pyraoxystrobin |
| B-16 | one individualized compound I | Pyribencarb |
| B-17 | one individualized compound I | Trifloxystrobin |
| B-18 | one individualized compound I | Triclopyricarb/Chlorodincarb |
| B-19 | one individualized compound I | 2-[2-(2,5-dimethyl-phenoxymethyl)-phenyl]-3-methoxy-acrylic acid methyl ester |
| B-20 | one individualized compound I | 2-(2-(3-(2,6-dichlorophenyl)-1-methyl-allylideneaminooxymethyl)-phenyl)-2-methoxyimino-N-methyl-acetamide |
| B-21 | one individualized compound I | Cyazofamid |
| B-22 | one individualized compound I | Amisulbrom |
| B-23 | one individualized compound I | Benalaxyl |
| B-24 | one individualized compound I | Benalaxyl-M |
| B-25 | one individualized compound I | Benodanil |
| B-26 | one individualized compound I | Benzovindiflupyr |
| B-27 | one individualized compound I | Bixafen |
| B-28 | one individualized compound I | Boscalid |
| B-29 | one individualized compound I | Carboxin |
| B-30 | one individualized compound I | Fenfuram |
| B-31 | one individualized compound I | Fenhexamid |
| B-32 | one individualized compound I | Flutolanil |
| B-33 | one individualized compound I | Fluxapyroxad |
| B-34 | one individualized compound I | Furametpyr |
| B-35 | one individualized compound I | Isofetamid |
| B-36 | one individualized compound I | Isopyrazam |
| B-37 | one individualized compound I | Isotianil |
| B-38 | one individualized compound I | Kiralaxyl |
| B-39 | one individualized compound I | Mepronil |
| B-40 | one individualized compound I | Metalaxyl |
| B-41 | one individualized compound I | Metalaxyl-M |
| B-42 | one individualized compound I | Ofurace |
| B-43 | one individualized compound I | Oxadixyl |
| B-44 | one individualized compound I | Oxycarboxin |
| B-45 | one individualized compound I | Penflufen |
| B-46 | one individualized compound I | Penthiopyrad |
| B-47 | one individualized compound I | Sedaxane |
| B-48 | one individualized compound I | Tecloftalam |
| B-49 | one individualized compound I | Thifluzamide |
| B-50 | one individualized compound I | Tiadinil |
| B-51 | one individualized compound I | 2-Amino-4-methyl-thiazole-5-carboxylic acid anilide |
| B-52 | one individualized compound I | N-(4'-trifluoromethylthiobiphenyl-2-yl)-3-difluoromethyl-1-methyl-1H-pyrazole-4-carboxamide |
| B-53 | one individualized compound I | N-(2-(1,3,3-trimethyl-butyl)-phenyl)-1,3-dimethyl-5-fluoro-1H-pyrazole-4-carboxamide |
| B-54 | one individualized compound I | 3-(difluoromethyl)-1-methyl-N-(1,1,3-tri-methylindan-4-yl)pyrazole-4-carbox-amide |
| B-55 | one individualized compound I | 3-(trifluoromethyl)-1-methyl-N-(1,1,3-tri-methylindan-4-yl)pyrazole-4-carboxamide |
| B-56 | one individualized compound I | 1,3-dimethyl-N-(1,1,3-trimethylindan-4-yl)pyrazole-4-carboxamide |
| B-57 | one individualized compound I | 3-(trifluoromethyl)-1,5-dimethyl-N-(1,1,3-trimethylindan-4-yl)pyrazole-4-carboxamide |
| B-58 | one individualized compound I | N-(7-fluoro-1,1,3-trimethyl-indan-4-yl)-1,3-dimethyl-pyrazole-4-carboxamide |
| B-59 | one individualized compound I | 1,3,5-trimethyl-N-(1,1,3-trimethylindan-4-yl)pyrazole-4-carboxamide |
| B-60 | one individualized compound I | N-[2-(2,4-dichlorophenyl)-2-methoxy-1-methyl-ethyl]-3-(difluoromethyl)-1-methyl-pyrazole-4-carboxamide |
| B-61 | one individualized compound I | Dimethomorph |
| B-62 | one individualized compound I | Flumorph |
| B-63 | one individualized compound I | Pyrimorph |
| B-64 | one individualized compound I | Flumetover |
| B-65 | one individualized compound I | Fluopicolide |
| B-66 | one individualized compound I | Fluopyram |
| B-67 | one individualized compound I | Zoxamide |
| B-68 | one individualized compound I | Carpropamid |
| B-69 | one individualized compound I | Diclocymet |
| B-70 | one individualized compound I | Mandipropamid |
| B-71 | one individualized compound I | Oxytetracyclin |
| B-72 | one individualized compound I | Silthiofam |
| B-73 | one individualized compound I | N-(6-methoxy-pyridin-3-yl)cyclopro-panecarboxylic acid amide |
| B-74 | one individualized compound I | Azaconazole |
| B-75 | one individualized compound I | Bitertanol |
| B-76 | one individualized compound I | Bromuconazole |
| B-77 | one individualized compound I | Cyproconazole |
| B-78 | one individualized compound I | Difenoconazole |
| B-79 | one individualized compound I | Diniconazole |
| B-80 | one individualized compound I | Diniconazole-M |
| B-81 | one individualized compound I | Epoxiconazole |
| B-82 | one individualized compound I | Fenbuconazole |
| B-83 | one individualized compound I | Fluquinconazole |
| B-84 | one individualized compound I | Flusilazole |
| B-85 | one individualized compound I | Flutriafol |
| B-86 | one individualized compound I | Hexaconazol |
| B-87 | one individualized compound I | Imibenconazole |
| B-88 | one individualized compound I | Ipconazole |
| B-89 | one individualized compound I | Metconazole |
| B-90 | one individualized compound I | Myclobutanil |
| B-91 | one individualized compound I | Oxpoconazol |
| B-92 | one individualized compound I | Paclobutrazol |
| B-93 | one individualized compound I | Penconazole |
| B-94 | one individualized compound I | Propiconazole |
| B-95 | one individualized compound I | Prothioconazole |
| B-96 | one individualized compound I | Simeconazole |
| B-97 | one individualized compound I | Tebuconazole |
| B-98 | one individualized compound I | Tetraconazole |
| B-99 | one individualized compound I | Triadimefon |
| B-100 | one individualized compound I | Triadimenol |
| B-101 | one individualized compound I | Triticonazole |
| B-102 | one individualized compound I | Uniconazole |
| B-103 | one individualized compound I | 1-[*rel*-(2S;3R)-3-(2-chlorophenyl)-2-(2,4-difluorophenyl)-oxiranylmethyl]-5-thiocyanato-1H-[1,2,4]triazole, |
| B-104 | one individualized compound I | 2-[*rel*-(2S;3R)-3-(2-chlorophenyl)-2-(2,4-difluorophenyl)-oxiranylmethyl]-2H-[1,2,4]triazole-3-thiol |
| B-105 | one individualized compound I | 2-[2-chloro-4-(4-chlorophenoxy)phenyl]-1-(1,2,4-triazol-1-yl)pentan-2-ol |
| B-106 | one individualized compound I | 1-[4-(4-chlorophenoxy)-2-(trifluorometh-yl)phenyl]-1-cyclopropyl-2-(1,2,4-triazol-1-yl)ethanol |
| B-107 | one individualized compound I | 2-[4-(4-chlorophenoxy)-2-(trifluorometh-yl)phenyl]-1-(1,2,4-triazol-1-yl)butan-2-ol |
| B-108 | one individualized compound I | 2-[2-chloro-4-(4-chlorophenoxy)phenyl]-1-(1,2,4-triazol-1-yl)butan-2-ol |
| B-109 | one individualized compound I | 2-[4-(4-chlorophenoxy)-2-(trifluorometh-yl)phenyl]-3-methyl-1-(1,2,4-triazol-1-yl)butan-2-ol |
| B-110 | one individualized compound I | 2-[4-(4-chlorophenoxy)-2-(trifluoromethyl)phenyl]-1-(1,2,4-triazol-1-yl)propan-2-ol |
| B-111 | one individualized compound I | 2-[2-chloro-4-(4-chlorophenoxy)phenyl]-3-methyl-1-(1,2,4-triazol-1-yl)butan-2-ol |
| B-112 | one individualized compound I | 2-[4-(4-chlorophenoxy)-2-(trifluoromethyl)phenyl]-1-(1,2,4-triazol-1-yl)pentan-2-ol; |
| B-113 | one individualized compound I | 2-[4-(4-fluorophenoxy)-2-(trifluoromethyl)phenyl]-1-(1,2,4-triazol-1-yl)propan-2-ol |
| B-114 | one individualized compound I | Amisulbrom |
| B-115 | one individualized compound I | Imazalil |
| B-116 | one individualized compound I | Imazalil-sulfate |
| B-117 | one individualized compound I | Pefurazoate |
| B-118 | one individualized compound I | Prochloraz |
| B-119 | one individualized compound I | Triflumizole |
| B-120 | one individualized compound I | Benomyl |
| B-121 | one individualized compound I | Carbendazim |
| B-122 | one individualized compound I | Fuberidazole |
| B-123 | one individualized compound I | Thiabendazole |
| B-124 | one individualized compound I | Ethaboxam |
| B-125 | one individualized compound I | Etridiazole |
| B-126 | one individualized compound I | Hymexazole |
| B-127 | one individualized compound I | 2-(4-Chloro-phenyl)-N-[4-(3,4-dimeth-oxy-phenyl)-isoxazol-5-yl]-2-prop-2-yn-yloxy-acetamide |
| B-128 | one individualized compound I | Fluazinam |
| B-129 | one individualized compound I | Pyrifenox |
| B-130 | one individualized compound I | 3-[5-(4-Chloro-phenyl)-2,3-dimethyl-is-oxazolidin-3-yl]-pyridine (Pyrisoxazole) |
| B-131 | one individualized compound I | 3-[5-(4-Methyl-phenyl)-2,3-dimethyl-isoxazolidin-3-yl]-pyridine |
| B-132 | one individualized compound I | Bupirimate |
| B-133 | one individualized compound I | Cyprodinil |
| B-134 | one individualized compound I | 5-Fluorocytosine |
| B-135 | one individualized compound I | 5-Fluoro-2-(p-tolylmethoxy)pyrimidin-4-amine |
| B-136 | one individualized compound I | 5-Fluoro-2-(4-fluorophenylmethoxy)-pyrimidin-4-amine |
| B-137 | one individualized compound I | Diflumetorim |
| B-138 | one individualized compound I | (5,8-Difluoroquinazolin-4-yl)-{2-[2-fluo-ro-4-(4-trifluoromethylpyridin-2-yloxy)-phenyl]-ethyl}-amine |
| B-139 | one individualized compound I | Fenarimol |
| B-140 | one individualized compound I | Ferimzone |
| B-141 | one individualized compound I | Mepanipyrim |
| B-142 | one individualized compound I | Nitrapyrin |
| B-143 | one individualized compound I | Nuarimol |
| B-144 | one individualized compound I | Pyrimethanil |
| B-145 | one individualized compound I | Triforine |
| B-146 | one individualized compound I | Fenpiclonil |
| B-147 | one individualized compound I | Fludioxonil |
| B-148 | one individualized compound I | Aldimorph |
| B-149 | one individualized compound I | Dodemorph |
| B-150 | one individualized compound I | Dodemorph-acetate |
| B-151 | one individualized compound I | Fenpropimorph |
| B-152 | one individualized compound I | Tridemorph |
| B-153 | one individualized compound I | Fenpropidin |
| B-154 | one individualized compound I | Fluoroimid |
| B-155 | one individualized compound I | Iprodione |
| B-156 | one individualized compound I | Procymidone |
| B-157 | one individualized compound I | Vinclozolin |
| B-158 | one individualized compound I | Famoxadone |
| B-159 | one individualized compound I | Fenamidone |
| B-160 | one individualized compound I | Flutianil |
| B-161 | one individualized compound I | Octhilinone |
| B-162 | one individualized compound I | Probenazole |
| B-163 | one individualized compound I | Fenpyrazamine |
| B-164 | one individualized compound I | Acibenzolar-S-methyl |
| B-165 | one individualized compound I | Ametoctradin |
| B-166 | one individualized compound I | Amisulbrom |
| B-167 | one individualized compound I | [(3S,6S,7R,8R)-8-benzyl-3-[(3-isobuty-ryloxymethoxy-4-methoxypyridine-2-carbonyl)amino]-6-methyl-4,9-dioxo-[1,5]dioxonan-7-yl]2-methylpropanoate |
| B-168 | one individualized compound I | [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate |
| B-169 | one individualized compound I | [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate |
| B-170 | one individualized compound I | [(3S,6S,7R,8R)-8-benzyl-3-[(3-isobut-oxycarbonyloxy-4-methoxy-pyridine-2-carbonyl)amino]-6-methyl-4,9-dioxo-1,5-dioxonan-7-yl] 2-methylpropanoate |
| B-171 | one individualized compound I | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methyl-propanoate |
| B-172 | one individualized compound I | (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate |
| B-173 | one individualized compound I | Anilazin |
| B-174 | one individualized compound I | Blasticidin-S |
| B-175 | one individualized compound I | Captafol |
| B-176 | one individualized compound I | Captan |
| B-177 | one individualized compound I | Chinomethionat |
| B-178 | one individualized compound I | Dazomet |
| B-179 | one individualized compound I | Debacarb |
| B-180 | one individualized compound I | Diclomezine |
| B-181 | one individualized compound I | Difenzoquat, |
| B-182 | one individualized compound I | Difenzoquat-methylsulfate |
| B-183 | one individualized compound I | Fenoxanil |
| B-184 | one individualized compound I | Folpet |
| B-185 | one individualized compound I | Oxolinsäure |
| B-186 | one individualized compound I | Piperalin |
| B-187 | one individualized compound I | Proquinazid |
| B-188 | one individualized compound I | Pyroquilon |
| B-189 | one individualized compound I | Quinoxyfen |
| B-190 | one individualized compound I | Triazoxid |
| B-191 | one individualized compound I | Tricyclazole |
| B-192 | one individualized compound I | 2-Butoxy-6-iodo-3-propyl-chromen-4-one |
| B-193 | one individualized compound I | 5-Chloro-1-(4,6-dimethoxy-pyrimidin-2-yl)-2-methyl-1H-benzoimidazole |
| B-194 | one individualized compound I | 5-Chloro-7-(4-methyl-piperidin-1-yl)-6-(2,4,6-trifluoro-phenyl)-[1,2,4]tri-azolo[1,5-a]pyrimidine |
| B-195 | one individualized compound I | Ferbam |
| B-196 | one individualized compound I | Mancozeb |
| B-197 | one individualized compound I | Maneb |
| B-198 | one individualized compound I | Metam |
| B-199 | one individualized compound I | Methasulphocarb |
| B-200 | one individualized compound I | Metiram |
| B-201 | one individualized compound I | Propineb |
| B-202 | one individualized compound I | Thiram |
| B-203 | one individualized compound I | Zineb |
| B-204 | one individualized compound I | Ziram |
| B-205 | one individualized compound I | Diethofencarb |
| B-206 | one individualized compound I | Benthiavalicarb |
| B-207 | one individualized compound I | Iprovalicarb |
| B-208 | one individualized compound I | Propamocarb |
| B-209 | one individualized compound I | Propamocarb hydrochlorid |
| B-210 | one individualized compound I | Valifenalate |
| B-211 | one individualized compound I | N-(1-(1-(4-cyanophenyl)ethanesulfon-yl)-but-2-yl) carbamic acid-(4-fluoro-phenyl) ester |
| B-212 | one individualized compound I | Dodine |
| B-213 | one individualized compound I | Dodine free base |
| B-214 | one individualized compound I | Guazatine |
| B-215 | one individualized compound I | Guazatine-acetate |
| B-216 | one individualized compound I | Iminoctadine |
| B-217 | one individualized compound I | Iminoctadine-triacetate |
| B-218 | one individualized compound I | Iminoctadine-tris(albesilate) |
| B-219 | one individualized compound I | Kasugamycin |
| B-220 | one individualized compound I | Kasugamycin-hydrochloride-hydrate |
| B-221 | one individualized compound I | Polyoxine |
| B-222 | one individualized compound I | Streptomycin |
| B-223 | one individualized compound I | Validamycin A |
| B-224 | one individualized compound I | Binapacryl |
| B-225 | one individualized compound I | Dicloran |
| B-226 | one individualized compound I | Dinobuton |
| B-227 | one individualized compound I | Dinocap |
| B-228 | one individualized compound I | Nitrothal-isopropyl |
| B-229 | one individualized compound I | Tecnazen |
| B-230 | one individualized compound I | Fentin salts |
| B-231 | one individualized compound I | Dithianon |
| B-232 | one individualized compound I | 2,6-dimethyl-1H,5H-[1,4]dithiino [2,3-c:5,6-c']dipyrrole-1,3,5,7(2H,6H)-tetraone |
| B-233 | one individualized compound I | Isoprothiolane |
| B-234 | one individualized compound I | Edifenphos |
| B-235 | one individualized compound I | Fosetyl, Fosetyl-aluminium |
| B-236 | one individualized compound I | Iprobenfos |
| B-237 | one individualized compound I | Phosphorous acid (H₃PO₃) and derivatives |
| B-238 | one individualized compound I | Pyrazophos |
| B-239 | one individualized compound I | Tolclofos-methyl |
| B-240 | one individualized compound I | Chlorothalonil |
| B-241 | one individualized compound I | Dichlofluanid |
| B-242 | one individualized compound I | Dichlorophen |
| B-243 | one individualized compound I | Flusulfamide |
| B-244 | one individualized compound I | Hexachlorbenzene |
| B-245 | one individualized compound I | Pencycuron |
| B-246 | one individualized compound I | Pentachlorophenol and salts |
| B-247 | one individualized compound I | Phthalide |
| B-248 | one individualized compound I | Quintozene |
| B-249 | one individualized compound I | Thiophanate Methyl |
| B-250 | one individualized compound I | Tolylfluanid |
| B-251 | one individualized compound I | N-(4-chloro-2-nitro-phenyl)-N-ethyl-4-methyl-benzenesulfonamide |
| B-252 | one individualized compound I | Bordeaux mixture |
| B-253 | one individualized compound I | Copper acetate |
| B-254 | one individualized compound I | Copper hydroxide |
| B-255 | one individualized compound I | Copper oxychloride |
| B-256 | one individualized compound I | basic Copper sulfate |
| B-257 | one individualized compound I | Sulfur |
| B-258 | one individualized compound I | Biphenyl |
| B-259 | one individualized compound I | Bronopol |
| B-260 | one individualized compound I | Cyflufenamid |
| B-261 | one individualized compound I | Cymoxanil |
| B-262 | one individualized compound I | Diphenylamin |
| B-263 | one individualized compound I | Metrafenone |
| B-264 | one individualized compound I | Pyriofenone |
| B-265 | one individualized compound I | Mildiomycin |
| B-266 | one individualized compound I | Oxin-copper |
| B-267 | one individualized compound I | Oxathiapiprolin |
| B-268 | one individualized compound I | Tolprocarb |
| B-269 | one individualized compound I | 2-[3,5-bis(difluoromethyl)-H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone |
| B-270 | one individualized compound I | 2-[3,5-bis(difluoromethyl)-1 H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]-ethanone |
| B-271 | one individualized compound I | 2-[3,5-bis(difluoromethyl)-1 H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]-ethanone |
| B-272 | one individualized compound I | ethyl (Z)-3-amino-2-cyano-3-phenyl-prop-2-enoate |
| B-273 | one individualized compound I | tert-butyl N-[6-[[(Z)-[(1-methyltetrazol-5-yl)-phenyl-methylene]amino]oxy-methyl]-2-pyridyl]carbamate |
| B-274 | one individualized compound I | pentyl N-[6-[[(Z)-[(1-methyltetrazol-5-yl)-phenyl-methylene]amino]oxy-methyl]-2-pyridyl]carbamate |
| B-275 | one individualized compound I | 2-[2-[(7,8-difluoro-2-methyl-3-quinol-yl)oxy]-6-fluoro-phenyl]propan-2-ol |
| B-276 | one individualized compound I | 2-[2-fluoro-6-[(8-fluoro-2-methyl-3-quinolyl)oxy]phenyl]propan-2-ol |
| B-277 | one individualized compound I | 3-(5-fluoro-3,3,4,4-tetramethyl-3,4-di-hydroisoquinolin-1-yl)quinoline |
| B-278 | one individualized compound I | 3-(4,4-difluoro-3,3-dimethyl-3,4-di-hydroisoquinolin-1-yl)quinoline |
| B-279 | one individualized compound I | 3-(4,4,5-trifluoro-3,3-dimethyl-3,4-di-hydroisoquinolin-1-yl)quinoline |
| B-280 | one individualized compound I | Prohexadione calcium |
| B-281 | one individualized compound I | Spiroxamine |
| B-282 | one individualized compound I | Tebufloquin |
| B-283 | one individualized compound I | Tolylfluanid |
| B-284 | one individualized compound I | N-(Cyclopropylmethoxyimino-(6-difluoromethoxy-2,3-difluoro-phenyl)-methyl)-2-phenyl acetamide |
| B-285 | one individualized compound I | N'-(4-(4-chloro-3-trifluoromethyl-phenoxy)-2,5-dimethyl-phenyl)-N-ethyl-N-methyl formamidine |
| B-286 | one individualized compound I | N'-(4-(4-fluoro-3-trifluoromethyl-phenoxy)-2,5-dimethyl-phenyl)-N-ethyl-N-methyl formamidine |
| B-287 | one individualized compound I | N'-(2-methyl-5-trifluoromethyl-4-(3-tri-methylsilanyl-propoxy)-phenyl)-N-ethyl-N-methyl formamidine |
| B-288 | one individualized compound I | N'-(5-difluoromethyl-2-methyl-4-(3-tri-methylsilanyl-propoxy)-phenyl)-N-ethyl-N-methyl formamidine |
| B-289 | one individualized compound I | Methoxy-acetic acid 6-tert-butyl-8-fluoro-2,3-dimethyl-quinolin-4-yl ester |
| B-290 | one individualized compound I | *Bacillus subtilis* NRRL No. B-21661 |
| B-291 | one individualized compound I | *Bacillus pumilus* NRRL No. B-30087 |
| B-292 | one individualized compound I | *Ulocladium oudemansii* |
| B-293 | one individualized compound I | Carbaryl |
| B-294 | one individualized compound I | Carbofuran |
| B-295 | one individualized compound I | Carbosulfan |
| B-296 | one individualized compound I | Methomylthiodicarb |
| B-297 | one individualized compound I | Bifenthrin |
| B-298 | one individualized compound I | Cyfluthrin |
| B-299 | one individualized compound I | Cypermethrin |
| B-300 | one individualized compound I | alpha-Cypermethrin |
| B-301 | one individualized compound I | zeta-Cypermethrin |
| B-302 | one individualized compound I | Deltamethrin |
| B-303 | one individualized compound I | Esfenvalerate |
| B-304 | one individualized compound I | Lambda-cyhalothrin |
| B-305 | one individualized compound I | Permethrin |
| B-306 | one individualized compound I | Tefluthrin |
| B-307 | one individualized compound I | Diflubenzuron |
| B-308 | one individualized compound I | Flufenoxuron |
| B-309 | one individualized compound I | Lufenuron |
| B-310 | one individualized compound I | Teflubenzuron |
| B-311 | one individualized compound I | Spirotetramate |
| B-312 | one individualized compound I | Clothianidin |
| B-313 | one individualized compound I | Dinotefuran |
| B-314 | one individualized compound I | Imidacloprid |
| B-315 | one individualized compound I | Thiamethoxam |
| B-316 | one individualized compound I | Flupyradifurone |
| B-317 | one individualized compound I | Acetamiprid |
| B-318 | one individualized compound I | Thiacloprid |
| B-319 | one individualized compound I | Endosulfan |
| B-320 | one individualized compound I | Fipronil |
| B-321 | one individualized compound I | Abamectin |
| B-322 | one individualized compound I | Emamectin |
| B-323 | one individualized compound I | Spinosad |
| B-324 | one individualized compound I | Spinetoram |
| B-325 | one individualized compound I | Hydramethylnon |
| B-326 | one individualized compound I | Chlorfenapyr |
| B-327 | one individualized compound I | Fenbutatin oxide |
| B-328 | one individualized compound I | Indoxacarb |
| B-329 | one individualized compound I | Metaflumizone |
| B-330 | one individualized compound I | Flonicamid |
| B-331 | one individualized compound I | Flubendiamide |
| B-332 | one individualized compound I | Chlorantraniliprole |
| B-333 | one individualized compound I | Cyantraniliprole |
| B-334 | one individualized compound I | N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide |
| B-335 | one individualized compound I | N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide |
| B-336 | one individualized compound I | N-[4-chloro-2-[(di-2-propyl-lambda-4-sulfanylidene)carbamoyl]-6-methyl-phenyl]-2-(3-chloro-2-pyridyl)-5-(tri-fluoromethyl)pyrazole-3-carboxamide |
| B-337 | one individualized compound I | N-[4,6-dichloro-2-[(di-2-propyl-lambda-4-sulfanylidene)carbamoyl]-phenyl]-2-(3-chloro-2-pyridyl)-5-(trifluoro-methyl)pyrazole-3-carboxamide |
| B-338 | one individualized compound I | N-[4,6-dichloro-2-[(diethyl-lambda-4-sulfanylidene)carbamoyl]-phenyl]-2-(3-chloro-2-pyridyl)-5-(difluoro-methyl)pyrazole-3-carboxamide |
| B-339 | one individualized compound I | N-[4,6-dibromo-2-[(di-2-propyl-lambda-4-sulfanylidene)carbamoyl]-phenyl]-2-(3-chloro-2-pyridyl)-5-(trifluoromethyl)-pyrazole-3-carboxamide |
| B-340 | one individualized compound I | N-[4-chloro-2-[(di-2-propyl-lambda-4-sulfanylidene)carbamoyl]-6-cyano-phenyl]-2-(3-chloro-2-pyridyl)-5-(tri-fluoromethyl)pyrazole-3-carboxamide |
| B-341 | one individualized compound I | N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide |
| B-342 | one individualized compound I | Cyflumetofen |
| B-343 | one individualized compound I | Acetochlor |
| B-344 | one individualized compound I | Dimethenamid |
| B-345 | one individualized compound I | metolachlor |
| B-346 | one individualized compound I | Metazachlor |
| B-347 | one individualized compound I | Glyphosate |
| B-348 | one individualized compound I | Glufosinate |
| B-349 | one individualized compound I | Sulfosate |
| B-350 | one individualized compound I | Clodinafop |
| B-351 | one individualized compound I | Fenoxaprop |
| B-352 | one individualized compound I | Fluazifop |
| B-353 | one individualized compound I | Haloxyfop |
| B-354 | one individualized compound I | Paraquat |
| B-355 | one individualized compound I | Phenmedipham |
| B-356 | one individualized compound I | Clethodim |
| B-357 | one individualized compound I | Cycloxydim |
| B-358 | one individualized compound I | Profoxydim |
| B-359 | one individualized compound I | Sethoxydim |
| B-360 | one individualized compound I | Tepraloxydim |
| B-361 | one individualized compound I | Pendimethalin |
| B-362 | one individualized compound I | Prodiamine |
| B-363 | one individualized compound I | Trifluralin |
| B-364 | one individualized compound I | Acifluorfen |
| B-365 | one individualized compound I | Bromoxynil |
| B-366 | one individualized compound I | I mazamethabenz |
| B-367 | one individualized compound I | Imazamox |
| B-368 | one individualized compound I | Imazapic |
| B-369 | one individualized compound I | Imazapyr |
| B-370 | one individualized compound I | Imazaquin |
| B-371 | one individualized compound I | Imazethapyr |
| B-372 | one individualized compound I | 2,4-Dichlorophenoxyacetic acid (2,4-D) |
| B-373 | one individualized compound I | Chloridazon |
| B-374 | one individualized compound I | Clopyralid |
| B-375 | one individualized compound I | Fluroxypyr |
| B-376 | one individualized compound I | Picloram |
| B-377 | one individualized compound I | Picolinafen |
| B-378 | one individualized compound I | Bensulfuron |
| B-379 | one individualized compound I | Chlorimuron-ethyl |
| B-380 | one individualized compound I | Cyclosulfamuron |
| B-381 | one individualized compound I | Iodosulfuron |
| B-382 | one individualized compound I | Mesosulfuron |
| B-383 | one individualized compound I | Metsulfuron-methyl |
| B-384 | one individualized compound I | Nicosulfuron |
| B-385 | one individualized compound I | Rimsulfuron |
| B-386 | one individualized compound I | Triflusulfuron |
| B-387 | one individualized compound I | Atrazine |
| B-388 | one individualized compound I | Hexazinone |
| B-389 | one individualized compound I | Diuron |
| B-390 | one individualized compound I | Florasulam |
| B-391 | one individualized compound I | Pyroxasulfone |
| B-392 | one individualized compound I | Bentazone |
| B-393 | one individualized compound I | Cinidon-ethyl |
| B-394 | one individualized compound I | Cinmethylin |
| B-395 | one individualized compound I | Dicamba |
| B-396 | one individualized compound I | Diflufenzopyr |
| B-397 | one individualized compound I | Quinclorac |
| B-398 | one individualized compound I | Quinmerac |
| B-399 | one individualized compound I | Mesotrione |
| B-400 | one individualized compound I | Saflufenacil |
| B-401 | one individualized compound I | Topramezone |
| B-402 | one individualized compound I | 1,1'-[(3S,4R,4aR,6S,6aS,12R,12aS, 12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11 H-naph-tho[2,1-b]pyrano[3,4-e]pyran-3,6-diyl] cyclopropaneacetic acid ester |

The active substances referred to as component 2, their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP-A141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, WO2012/168188, WO 2007/006670, WO 2011/77514; WO13/047749, WO 10/069882, WO 13/047441, WO 03/16303, WO 09/90181, WO 13/007767, WO 13/010862, PCT/EP2012/065650 and PCT/EP2012/065651).

The mixtures of active substances can be prepared as compositions comprising besides the active ingridients at least one inert ingredient by usual means, e. g. by the means given for the compositions of compounds I.

Concerning usual ingredients of such compositions reference is made to the explanations given for the compositions containing compounds I.

The mixtures of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

### I. Synthesis examples

With due modification of the starting compounds, the procedures shown in the descirptionwere used to obtain compounds I. The resulting compounds, together with physical data, are listed in Table I below.

**Table I. Compounds of formula I**

| **No.** | **R¹** | **R²** | **R^{C}-R^{B}-R³** | **R⁴** | **X** | **Y** | **¹H-NMR (δ)** |
|---|---|---|---|---|---|---|---|
| I-1 | CH₃ | H | R3-2 | R4-1, X = NH | O | Y-1 | δ = 2.50 (s) 2.67 (m), 3.32 (s), 43.91 (s), 4.46 (m), 5.83 (m), 6.08 (m), 7.78 (d), 7.87 (d), 8.15 (s), 8.45 (s) |

### II. Examples of the action against harmful fungi

The fungicidal action of the compounds of the formula I was demonstrated by the following experiments:

### II.1 Microtiter tests

The active substances were formulated separately as a stock solution in dimethyl sulfoxide (DMSO) at a concentration of 10 000 ppm.

### Use example 1: Activity against the Septoria blotch pathogen caused by Septoria tritici in the microtiter test

Fungal strains used:
a) Septoria tritici (Qo-inhibitor sensitive, wild-type)
b) Septoria tritici (Qo-inhibitor-resistant, G143A mutant)

100 ml 2 % Malt extract in water at pH6.8 were inoculated with microspores from 2 week old cultures grown on 2 % malt extract + 2 % agar in Petri dishes and incubated for 3 days on a rotary shaker at 24°C und 150 rpm. The culture was harvested, glycerol was added (15 % (v/v) and kept frozen at -20°C in aliquots of 1 ml.

1ml stock suspension was thawed and suspended into 800 ml of 2 % malt extract in water at pH 6.8. Compounds were diluted from stock solution in (dimethylsulfoxide) DMSO in 10 steps. The compound solutions were diluted 1/5 with sterile deionized water before use. 5 µl of the compound solutions were transferred into empty microplates. The plates were then filled with 195 µl of the microspore suspension of each strain.

The antifungal activity was determined by measuring the turbidity of a culture in 96-well microplates in the presence of test compounds. Fungal growth was measured by recording the optical density at 620 nm every 15 h for 150 h.The relative antifungal activity was calculated by comparison of the effect of the test compounds with the effect of a DMSO control and a standard fungicide.

IC₅₀-values (concentration of test compound resulting in 50% inhibition of fungal growth) were calculated from the resulting dose-response for each compound and strain. The initial concentration of the test compounds and the 10 steps of dilution (1:4 each) allowed IC₅₀-values from 0.001 to 100 µmol/l (µM) to be assessed.

**Table II.**

| **Compound** | **Resistant *Septoria tritici* isolate (G143A mutation) (R-IC₅₀) [µM]** | **Sensitive *Septoria tritici* isolate (wild type) (S-IC₅₀) [µM]** | **Resistance factor RF = R-IC₅₀/S-IC₅₀** |
|---|---|---|---|
| Azoxystrobin | > 100 | 3.5 | n.d. |
| Dimoxystrobin | > 100 | 7.1 | n.d. |
| Enestroburin | > 100 | 4.5 | n.d. |
| Kresoximmethyl | > 100 | 0.76 | n.d. |
| Metominostrobin | > 100 | > 100 | n.d. |
| Orysastrobin | > 100 | 27 | n.d. |
| Picoxystrobin | > 100 | 2.3 | n.d. |
| Pyrametostrobin | > 100 | > 100 | n.d. |
| Pyraoxystrobin | > 100 | 1.6 | n.d. |
| Pyraclostrobin | 3.4 | 0.0012 | 2882 |
| Trifloxystrobin | > 100 | 0.52 | n.d. |
| | | | |
| I-1 | 3.7 | 1.0 | 3.7 |

While commercial strobilurine type fungicides are do not show any activity against the resistant Septoria strain, containing a mutation in the mitochondrial cytochrome b gene conferring resistance to Qo inhibitors (G143A) while being active against sensitive wild type strains, compounds I have been active against both, the resistant and the wild-type strains. In general, the resistance factor ratio (RF) calculated from the IC50 values determined for both Septoria strains, was below 30 for the compounds I according to the invention. However, resistance factor ratios for commercial strobilurine type compounds are in most cases greater than 100 and usually greater than several hundreds (for details see e.g. FRAC, Mutations associated with Qol-resistance, Dec. 2006; http://frac.info/frac/work/Mutations%20associated%20with%20Qo1%20resistance.pdf and citations cited therein).

### B) Glass house trials

The spray solutions were prepared in several steps:
The stock solution was prepared as follows: 1.26 ml of a 1:1 mixture of cyclohexanone and dimethylsulfoxide was added to 8.4 mg of active ingredient. Next, 40.74 ml of a mixture of water, acetone (10%), the emulsifier Wettol (0.1 %) and the wetting agent Silwet (0.05%) was added. This stock solution was then further diluted with the described solvent-emulsifier-water mixture to the desired concentrations.

### Trial 1: Preventative fungicidal control of Botrytis cinerea on leaves of green pepper

Young seedlings of green pepper were grown in pots to the 4 to 5 leaf stage. These plants were sprayed to run-off with an aqueous suspension, containing the concentration of active ingredient or their mixture mentioned in the table below. The next day the plants were inoculated with an aqueous biomalt solution containing the spore suspension of *Botrytis cinerea.* Then the plants were immediately transferred to a humid chamber. After 5 days at 22 to 24°C and a relative humidity close to 100% the extent of fungal attack on the leaves was visually assessed as % diseased leaf area.

In this test, the plants which had been treated with 100 ppm of the active compound from example I-1 showed an infection of 5 % whereas the untreated plants were 100% infected.

### Trial 2: Control of powdery mildew on wheat caused by Blumeria graminis f. sp. tritici

The first fully developed leaves of pot grown wheat were sprayed to run-off with an aqueous suspension, containing the concentration of active ingredient or their mixture mentioned in the table below. The next day the treated plants were inoculated with spores of *Blumeria graminis* f. sp. *tritici* (= syn. *Erysiphe garminis f.* sp. *tritici*) by shaking heavily infestated stock plants over the treated pots. After cultivation in the greenhouse for 7 days at 21-23°C and a relative humidity between 40 to 70% the extent of fungal attack on the leaves was visually assessed as % diseased leaf area.

In this test, the plants which had been treated with 100 ppm of the active compound from example I-1 showed an infection of 0 % whereas the untreated plants were 70% infected.

### Trial 3: Curative control of soy bean rust on soy beans caused by Phakopsora pachyrhizi

Leaves of pot-grown soy bean seedlings were inoculated with spores of *Phakopsora pachyrhizi.* To ensure the success of the artificial inoculation, the plants were transferred to a humid chamber with a relative humidity of about 95% and 20 to 24°C for 24 h. The next day the plants were sprayed to run-off with an aqueous suspension, containing the concentration of active ingredient or their mixture as described below. The plants were allowed to air-dry. Then the trial plants were cultivated for 14 days in a greenhouse chamber at 23-27°C and a relative humidity between 60 and 80%. The extent of fungal attack on the leaves was visually assessed as % diseased leaf area.

In this test, the plants which had been treated with 100 ppm of the active compound from example I-1 showed an infection of 0 % whereas the untreated plants were 90% infected.

### Trial 4: Control of late blight on tomatoes caused by Phytophthora infestans

Young seedlings of tomato plants were grown in pots. These plants were sprayed to run-off with an aqueous suspension, containing the concentration of active ingredient or their mixture mentioned in the table below. The next day, the treated plants were inoculated with an aqueous suspension of sporangia of *Phytophthora infestans.* After inoculation, the trial plants were immediately transferred to a humid chamber. After 6 days at 18 to 20°C and a relative humidity close to 100% the extent of fungal attack on the leaves was visually assessed as % diseased leaf area.

In this test, the plants which had been treated with 100 ppm of the active compound from example I-1 showed an infection of 0 % whereas the untreated plants were 90% infected.

### Trial 5: Preventative control of brown rust on wheat caused by Puccinia recondita

The first two developed leaves of pot-grown wheat seedling were sprayed to run-off with an aqueous suspension, containing the concentration of active ingredient or their mixture as described below. The next day the plants were inoculated with spores of *Puccinia recondita.* To ensure the success the artificial inoculation, the plants were transferred to a humid chamber without light and a relative humidity of 95 to 99% and 20 to 24°C for 24 h. Then the trial plants were cultivated for 6 days in a greenhouse chamber at 20-24°C and a relative humidity between 65 and 70%. The extent of fungal attack on the leaves was visually assessed as % diseased leaf area.

In this test, the plants which had been treated with 100 ppm of the active compound from example I-1 showed an infection of 0 % whereas the untreated plants were 90% infected.

### Trial 6: Preventative control of leaf blotch on wheat caused by Septoria tritici (Septtr P1)

Leaves of pot-grown wheat seedling were sprayed to run-off with an aqueous suspension of the active compound or their mixture, prepared as described. The plants were allowed to air-dry. At the following day the plants were inoculated with an aqueous spore suspension of *Septoria tritici.* Then the trial plants were immediately transferred to a humid chamber at 18-22°C and a relative humidity close to 100%. After 4 days the plants were transferred to a chamber with 18-22°C and a relative humidity close to 70%. After 4 weeks the extent of fungal attack on the leaves was visually assessed as % diseased leaf area.

In this test, the plants which had been treated with 100 ppm of the active compound from example I-1 showed an infection of 0 % whereas the untreated plants were 90% infected.

## Claims

1. Compounds of formula I 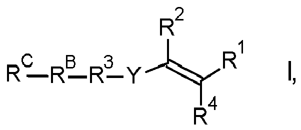 wherein:
R¹,R² independently of each other are hydrogen, halogen, C₁-C₄-alkyl, C₁-C₄-alkoxy, C₂-C₆-alkenyl, C₂-C₆-alkenyloxy, C₂-C₆-alkynyl, C₃-C₆-cycloalkyl or C₃-C₆-cycloalkyl-C₁-C₄-alkyl, wherein the groups R¹ and R² are cis-oriented, and
wherein the aliphatic moieties of R¹ and/or R² may carry 1, 2, 3 or up to the maximum number of identical or different groups R^{a} which independently of one another are selected from:
R^{a} halogen, CN, nitro, C₁-C₄-alkyl, C₁-C₄-alkoxy, C₁-C₄-haloalkyl and C₁-C₄-haloalkoxy;
Y is a direct bond or a divalent group selected from -OCH₂-, -CH₂- or-CH₂CH₂-, where the bond depicted on the left side of the divalent group Y is attached to R³, and the bond depicted on the right side is attached to the carbon atom being substituted by R²;
R³ is phenyl or a 3- to 10-membered saturated, partially unsaturated or aromatic mono- or bicyclic heterocyclyl wherein the ring member atoms of the heterocyclyl include besides carbon atoms 1, 2, 3 or 4 heteroatoms selected from the group of N, O and S,
wherein the group R³ may 1, 2, 3 or up to the maximum possible number of identical or different groups R^{b} which independently of one another are selected from:
R^{b} , which may be the same or different to any other R^{b}, is amino, halogen, hydroxyl, oxo, nitro, CN, carboxyl, C₁-C₄-alkyl, C₂-C₄-alkenyl, C₂-C₄-alkynyl, C₁-C₄-haloalkyl, C₁-C₄-alkoxy, C₁-C₄-haloalkoxy, C₃-C₆-cycloalkyl, C₃-C₆-cycloalkenyl, C₂-C₆-alkenyloxy, C₃-C₆-alkynyloxy, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₄-alkylamino, C₁-C₄-alkoxycarbonyl, C₁-C₄-alkylcarbonyloxy, phenyl, naphthyl or a 3- to 10-membered saturated, partially unsaturated or aromatic mono- or bicyclic heterocyclyl which, in addition to carbon atoms, contains one to four heteroatoms from the group consisting of N, O and S as ring members; and wherein the aforementioned phenyl and heterocyclyl groups R^{b} are attached via a direct bond, an oxygen or sulfur atom, and two radicals R^{b} that are bound to adjacent ring member atoms of the cyclic group R³ may form together with said ring member atoms a fused 5-, 6- or 7-membered saturated, partially unsaturated or aromatic cycle, which may be a carbocycle or heterocycle, wherein the ring member atoms of the heterocycle include besides carbon atoms 1, 2, 3 or 4 heteroatoms selected from the group of N, O and S;
R^{B} is phenyl or a 3- to 10-membered saturated, partially unsaturated or aromatic mono- or bicyclic heterocyclyl wherein the ring member atoms of the heterocyclyl include besides carbon atoms 1, 2, 3 or 4 heteroatoms selected from the group of N, O and S, wherein the group R^{B} may carry 1, 2, 3 or up to the maximum possible number of identical or different groups R^{CC}: R^{CC}, which may be the same or different to any other R^{CC}, is halogen, hydroxyl, nitro, CN, carboxyl, C₁-C₄-alkyl, C₂-C₄-alkenyl, C₂-C₈-alkynyl, C₁-C₄-haloalkyl, C₁-C₄-alkoxy, C₁-C₄-haloalkoxy, C₁-C₆-alkoxyimino-C₁-C₄-alkyl, C₂-C₆-alkenyloxyimino-C₁-C₄-alkyl , C₂-C₆-alkynyloxyimino-C₁-C₄-alkyl, C₁-C₆-alkoxyimino-, C₂-C₆-alkenyloxyimino-, C₂-C₆-alkynyloxyimino-, C₂-C₆-haloalkenyloxyimino-, -SR^{D}, -S(=O)R^{D}, S(=O)₂R^{D}, -N(R^{E})₂, -NH-C(=O)-N(R^{F})₂, -O-C(R^{F})-C(R^{F})=N-O-R^{F}, C₃-C₆-cycloalkyl, C₃-C₆-cycloalkenyl, phenyl or a 5-membered saturated, partially unsaturated or aromatic heterocyclyl which, in addition to carbon atoms, contains one to three heteroatoms from the group consisting of N, O and S as ring members; wherein the aforementioned cyclic groups R^{CC} are attached via a direct bond, an oxygen or sulfur atom, and where the aliphatic or cyclic groups R^{CC} for their part may carry 1, 2, 3 or up to the maximum possible number of identical or different groups R^{d}:
R^{d} , which may be the same or different to any other R^{d}, is halogen, C₁-C₄-alkyl or C₁-C₄-haloalkyl;
R^{C} is -SR^{D}, -S(=O)R^{D}, -S(=O)₂R^{D}, -N(R^{E})₂, -NH-C(=O)-N(R^{F})₂ or -O-C(R^{F})-C(R^{F})=N-O-R^{F}, wherein
R^{D} is C₁-C₄-alkyl, C₁-C₄-haloalkyl, C₁-C₄-alkoxy or C₁-C₄-haloalkoxy;
R^{E} , which may be the same or different to any other R^{E}, is hydrogen, C₁-C₄-alkyl, C₁-C₄-haloalkyl, C₁-C₄-alkylcarbonyl or C₁-C₄-haloalkyl-carbonyl;
R^{F} , which may be the same or different to any other R^{F}, is hydrogen, C₁-C₄-alkyl or C₁-C₄-haloalkyl;
R⁴ is a monovalent group selected from formulae R4-1 to R4-7 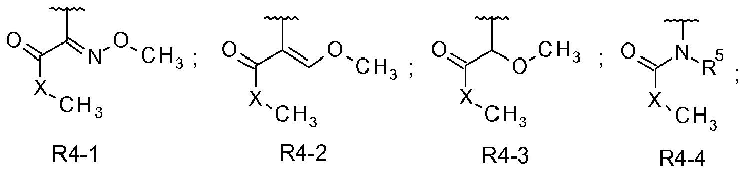 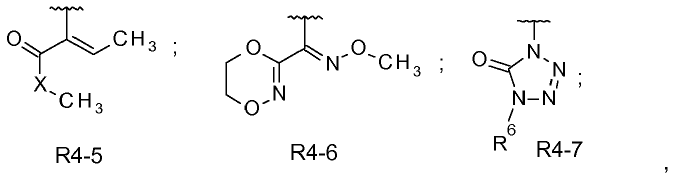
wherein the jagged line defines the point of attachment, and
X is a direct bond or a divalent group CH₂, O or NH,
R⁵ is C₁-C₄-alkyl, C₁-C₄-alkoxy, C₁-C₄-haloalkyl, C₁-C₄-haloalkoxy or C₃-C₆-cycloalkyl; and
R⁶ is C₁-C₄-alkyl or C₁-C₄-haloalkyl;
and the N-oxides and the agriculturally acceptable salts thereof.

2. The compounds according to claim 1, wherein R¹ is methyl or ethyl.

3. The compounds according to any of the claims 1 to 2, wherein R² is hydrogen.

4. The compounds according to any of the claims 1 to 3, wherein Y is -OCH₂-.

5. The compounds according to any of the claims 1 to 4, wherein R³ is pyrazolyl or 1,2,4-triazolyl.

6. The compounds according to any of the claims 1 to 5, wherein R^{B} is phenyl.

7. The compounds according to any of the claims 1 to 6, wherein R^{C} is -SR^{D}, -S(=O)R^{D} or -S(=O)₂R^{D}, wherein R^{D} is C₁-C₄-alkyl, C₁-C₄-haloalkyl, C₁-C₄-alkoxy or C₁-C₄-haloalkoxy.

8. The compounds according to any of the claims 1 to 7, wherein R⁴ is -C(=NOCH₃)-CONHCH₃, -C(=NOCH₃)-COOCH₃, -C(=CHOCH₃)-COOCH₃, -C(=CHOCH₃)-CONHCH₃, -N(OCH₃)-COOCH₃, -N(CH₃)-COOCH₃ or -N(CH₂CH₃)-COOCH₃.

9. Agrochemical compositions wherein said compositions comprise an auxiliary and at least one compound of formula I, as defined in any of the claims 1 to 8, an N-oxide or an agriculturally acceptable salt thereof.

10. The compositions according to claim 9 comprising at least one further active substance.

11. Use of compounds of the formula I and the N-oxides and the agriculturally acceptable salts thereof as defined in any of the claims 1 to 8 for combating phytopathogenic fungi.

12. The use according to claim 12 wherein the phytopathogenic fungi contain a mutation in the mitochondrial cytochrome b gene conferring resistance to Qo inhibitors, wherein the mutation is G143A.

13. A method for combating phytopathogenic fungi as defined in any of the claims 11 to 12, comprising:
treating the phytopathogenic fungi as defined in any of the claims 11 to 12 or the materials, plants, the soil or seeds that are at risk of being diseased from phytopathogenic fungi as defined in any of the claims 11 to 12 with an effective amount of at least one compound of formula I as defined in any of the claims 1 to 8, or with an effective amount of a composition as defined in any of claims 9 and 10.

14. The method according to claim 13, comprising:
a) identifying the phytopathogenic fungi as defined in any of the claims 11 to 12, or the materials, plants, the soil or seeds that are at risk of being diseased from phytopathogenic fungi as defined in any of the claims 11 to 12,
and
b) treating said fungi or the materials, plants, the soil or seeds with an effective amount of at least compound of formula I as defined in any of the claims 1 to 8, or with an effective amount of a composition as defined in any of claims 9 and 10.

15. Plant propagation material, comprising at least one compound of formula I as defined in any of the claims 1 to 8 in an amount of from 0.01 g to 10 kg per 100 kg of plant propagation material.
